(19) Europäisches Patentamt / European Patent Office / Office européen des brevets

(11) **EP 2 144 590 B1**

(12) **EUROPEAN PATENT SPECIFICATION**

(45) Date of publication and mention of the grant of the patent:
**01.08.2018 Bulletin 2018/31**

(21) Application number: **08754134.8**

(22) Date of filing: **28.04.2008**

(51) Int Cl.:
***A61K 8/9789*** *(2017.01)*
***A61K 8/34*** *(2006.01)*
***A61K 8/365*** *(2006.01)*
***A61Q 17/04*** *(2006.01)*
***A61Q 19/08*** *(2006.01)*
***A61Q 19/10*** *(2006.01)*
***A61K 9/00*** *(2006.01)*
***A61K 31/05*** *(2006.01)*
***A61K 36/487*** *(2006.01)*
***A61K 8/368*** *(2006.01)*

(86) International application number:
**PCT/US2008/005460**

(87) International publication number:
**WO 2008/140673 (20.11.2008 Gazette 2008/47)**

(54) **SKIN TREATMENT COMPOSITIONS AND METHODS**

HAUTBEHANDLUNGSZUSAMMENSETZUNGEN UND -VERFAHREN

COMPOSITIONS DE TRAITEMENT POUR LA PEAU ET PROCÉDÉS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **14.05.2007 US 803188**
**26.11.2007 US 986719**

(43) Date of publication of application:
**20.01.2010 Bulletin 2010/03**

(73) Proprietor: **Sytheon Ltd.**
**Lincoln Park, NJ 07035 (US)**

(72) Inventor: **CHAUDHURI, Ratan, K.**
**Lincoln Park, NJ 07035 (US)**

(74) Representative: **Tomkins & Co**
**5 Dartmouth Road**
**Dublin 6 (IE)**

(56) References cited:
**WO-A1-96/12487**
**DE-A1- 3 417 234**
**GB-A- 2 320 432**
**US-A1- 2001 001 666**
**US-A1- 2001 056 071**
**US-A1- 2002 086 039**
**US-A1- 2004 067 243**
**US-A1- 2005 048 008**
**US-A1- 2006 128 771**
**US-A1- 2006 251 749**
**US-A1- 2006 251 749**

- **ZANDER E ET AL: "TREATMENT OF ACNE VULGARIS WITH SALICYLIC ACID PADS", CLINICAL THERAPEUTICS, EXCERPTA MEDICA, PRINCETON, NJ, US, vol. 14, no. 2, 1 January 1992 (1992-01-01), pages 247-253, XP009176614, ISSN: 0149-2918**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 144 590 B1

## Description

### FIELD OF THE INVENTION

This invention relates to novel compositions and methods for treating skin wherein skin irritation and/or sun sensitivity resulting from the presence of dermatological acids, especially alpha- and/or beta- hydroxy acids, retinoic acids and the like, are reduced.

### BACKGROUND OF THE INVENTION

Dermatological acids are carboxylic acids that are incorporated into various therapeutic and non-therapeutic skin treatment compositions and products for purposes of aggressively or passively removing skin cells and/or cleaning the pores of the skin and/or for purposes of treating various dermatological conditions such as acne and the like.

Some of the more aggressive dermatological acid containing compositions and products are those known as chemical peels and are generally characterized as comprising a strong dermatological acid in relatively high concentrations and an appropriate carrier. Therapeutic chemical peels are the most aggressive and require professional, medical application. These compositions have a relatively deep penetration into the skin, causing the blistering and/or dissociation of skin layers deep in the skin whereby the dissociated layers may be removed to reveal a much newer, younger skin layer. Less aggressive chemical peels are also formulated for application by trained personnel at salons, spas and the like as well as for home, especially do-it-yourself treatments, whereby only the upper layers of the skin are removed. Dermatological acids especially suited for and commonly employed in chemical peels include various hydroxy acids including, in particular, alpha and beta hydroxy acids such as lactic acid, glycolic acid, salicylic acid; retinoic acids; trichloroacetic acid; and the like, as well as combinations thereof, especially Jessner's peel which comprises a combination of salicylic and lactic acids. As noted, the selection of the acid will depend upon its strength and the desired chemical peel, including the experience and training of the person administering the peel. With the weaker alpha-hydroxy acids, for example, the acid will be present at a level of 20-70%, with the chemical peel being applied for 15 to 20 minutes before it is neutralized and then removed by washing. Stronger acid chemical peels will have lower concentrations, e.g., 20-35% in the case of trichloroacetic acid, and will be applied for shorter periods of time, on the order of a few minutes, before being neutralized and removed.

Dermatological acids, including those mentioned above, also find themselves in a number of prescription and over-the-counter therapeutic compositions and products for the treatment of adverse skin conditions including, for example, acne, chloasma, psoriasis, calluses, corns, keratosis, and warts, especially acne. Depending upon the severity of the dermatological condition, more or less aggressive dermatological acids and/or higher or lower concentrations thereof may be used. Here, the objective is to remove the upper layers of skin, especially those embodying or afflicted with the adverse skin conditions to be addressed as well as to open pores and follicles that are clogged or blocked, i.e., dislodge or break down comedones, and/or keep clear those pores and follicles that have not yet become clogged or blocked. More aggressive compositions typically require a prescription and/or may be applied professionally, e.g., by a dermatologist; whereas, less aggressive or milder compositions are sold over-the-counter for personal use. Application is generally on a daily basis, if not multiple times each day, so long as the condition persists. As noted above, many if not all of the dermatological acids employable in chemical peels are also suitable for use in the treatment of acne and other adverse skin conditions. Particularly beneficial are the azelaic, salicylic and glycolic acids as well as the retinoids including, especially, adapalene, tretinoin, isotretinoin and tazarotene.

Weaker dermatological acids and lower concentrations of the more aggressive or moderately aggressive dermatological acids are also employed as constituents of various cosmetic compositions and products, particularly those products claiming to reduce wrinkles or the signs of aging and improve the overall look and feel of the skin, including skin cleansers and washes; skin-care creams, lotions, and powders; facial make-ups including bases and foundations, blushes, rouges, and the like; shampoos and other hair care products that also are to be worked into or applied to the scalp; and the like. The daily or repetitive use of these compositions helps with exfoliation and desquamation of dead skin cells, a natural process that is slowed with aging, as well as with the cleaning of pores and follicles, thereby preventing the formation of comedones and other adverse skin conditions.

While these dermatological acids have many beneficial attributes, as mentioned above, they are not without their adverse consequences. For example, salicylic acid, also known as 2-hydroxybenzoic acid, a key additive in many skin-care products, has been shown to be extremely efficacious in the treatment of acne and other skin diseases and adverse conditions. However, despite its safety as a food preservative at low concentrations, for those people with salicylate sensitivity, even small doses can be extremely harmful. Furthermore, the use of concentrated solutions of salicylic acid has been associated with the manifestation of hyperpigmentation on un-pretreated skin, particularly for those with darker skin types (Fitzpatrick phototypes IV, V, VI), as well as with the lack of or insufficient use of broad spectrum sunscreens. Also, salicylic acid tends to be somewhat drying and irritating and can often cause excessive

peeling, causing individuals to use the products less frequently and copiously than is necessary to obtain an optimum benefit.

Generally speaking, hydroxy acids, especially the alpha hydroxy acids and beta hydroxy acids, are associated with number of physiological effects on skin including the break down of the intercellular bonds or glue-like substance that binds to dead skin cells, the removal of impurities and accumulated cell debris from the skin surface, the promotion of collagen production, a reduction in the appearance of age spots, and a moisturizing action. Despite these benefits, hydroxy acids are also associated with skin irritation, inflammation, redness, and flaking: the severity of which, in part, is dependent upon the pH and the concentration of the acid used. In high concentrations, especially in professional chemical peels, they have more severe side-effects including blistering, burning, pain, and skin discoloration. Furthermore, an industry-sponsored study has now found that the use of hydroxy acids is associated with an increased photosensitivity to the sun.

Such adverse consequences are not limited to the hydroxy acids. For example, retinoic acid (Retin A), another common ingredient for skin treatment compositions, is widely used for acne treatment. Retinoic acid affects cell function and results in a loosening of the cells at the surface of the skin. However, serious concerns exist regarding the possibility of retinoic acid treatments playing a role in the development of skin cancers. In an experimental model, topical application of retinoic acid augmented photocarcinogenesis by reducing the latency period, increasing the total numbers of tumors, and decreasing survival time (Halliday, G. M. et al., Topical retinoic acid enhances, and a dark tan protects, from subdermal solar-simulated photocarcinogenesis. 114 J. Invest. Dermatol. 923 (2000).

While there are alternatives to the dermatological acids, they too are not without adversity. For example phenol peels (phenol and croton oil) are oftentimes used to provide a chemical peel, and are one of the most aggressive forms of chemical peels. Indeed, phenol peels are so aggressive that sedation if not outright application of general anesthesia is required owing to the pain associated with this treatment. Furthermore, recovery from the skin damage due to phenol peels is long in coming, oftentimes requiring several months to heal, and there is growing evidence of adverse heart conditions and nerve damage associated with phenol exposure. US2005048008 describes bakuchiol as an anti-inflammatory agent and an active ingredient in the treatment of COX ad LOX mediated diseases and conditions of the skin, in particular skin hyper-pigmentation, age spots, acne and the like. Similarly, various antibiotics may be employed to treat acne; however, antibiotic treatment is non-discriminatory and good bacteria as well as those causing the acne are attacked. Furthermore, the long-term use of antibiotics can lead to resistance to those antibiotics and/or reduce the body's resistance to attack by pathogenic bacterial that are otherwise held in check by good bacteria. Similarly, hormonal therapy is also possible, but the long term administration of hormones can have various adverse consequences as well.

Thus, there is a continuing need for skin treatments and cosmetic products wherein adverse skin reactions to and enhanced UV sensitivity caused by dermatological acids present in such treatments and products are reduced, if not eliminated.

Similarly, there is a continuing need for skin treatments and cosmetic products wherein the presence or amount of dermatological acids may be reduced with minimal or negligible effect on the performance of the acids in achieving the purpose for which they are present in said treatments and products.

Now, therefore, in accordance with the practice of the present invention there are provided skin treatment compositions and cosmetic products which manifest reduced adverse skin reactions and/or UV sensitivity typically associated with the use of dermatological acids. Additionally, there are provided skin treatment compositions and cosmetic products wherein the performance of the dermatological acid is synergistically enhanced. Finally, there are provided skin treatment compositions and cosmetic products wherein overall skin appearance is improved as compared to the use of traditional products containing the same dermatological acids.

SUMMARY

According to the present invention there are provided skin treatment compositions and cosmetic products comprising a) salicylic acid; b) bakuchiol and (c) a dermatological acceptable carrier, wherein the bakuchiol (b) is (i) present as a purified or isolated material having a purity of at least 60% w/w or as a purified plant extract containing 1 to about 70% or more by weight bakuchiol based on the total weight of the extract and (ii) contains generally less than 0.1 wt% of furocoumarins and wherein the amount of bakuchiol present in the composition is at least its median inhibitory concentration on *Propionibacterium acne* (MIC) amount and the amount of salicylic acid present in the composition is at least twice its MIC amount. In a preferred embodiment, the bakuchinol is free or substantially free of coumarins, especially furocoumarins like psoralene and iso-psoralene, and other like compounds. In accordance with the present invention, the bakuchinol is present in an amount that is effective for reducing the adverse skin effects, most notably irritancy, inflammation and/or reddening of skin and/or skin sensitivity to UV light resulting from the use of the dermatological acid(s). The last required component of the inventive compositions and products is a dermatologically acceptable carrier that is suitable for the specific end use of the inventive compositions and products and is substantially non-reactive with either of the two aforementioned active components.

In another respect, the present invention pertains to therapeutic compositions for the treatment of acne, rosacea, and other skin disorders as well as methods for the treatment of the same. Specifically, there are provided acne treatments comprising a moderate to high level of salicylic acid and a bakuchinol together with a suitable carrier for applying the actives to the acne afflicted skin as well as a method of treating such afflicted skin which method comprises the repeated application of the therapeutic composition to the afflicted skin.

In another aspect of the present invention there are provided chemical peels for professional as well as non-professional and/or self-administered application for the aggressive and short term removal of the upper layer(s) of skin. Chemical peels will vary in the amount of the actives, especially the acid, depending upon the skill of the person applying the same. In following, there is also described a method of performing a chemical peel which method comprises applying the composition to the skin to be peeled, allowing the same to remain on the skin for a sufficient period of time to cause the upper layer(s) of the treated skin to dissociate, by blistering, exfoliation or desquamation, from the underlying skin layers, then washing the treatment away. Depending upon the aggressiveness of the chemical peel, all or essentially all of the stratum corneum may be removed and, in extreme cases, layers within the stratum lucidum, may also be removed.

Finally, in yet another aspect of the present invention there are provided cosmetic compositions and products for enhancing overall skin appearance and skin tone which compositions comprise lower levels of the salicylic acid and/or the presence of weak salicylic acids such that exfoliation/desquamation of the uppermost layers of the dead skin is enhanced with removal of the cosmetic product. Because these products are intended for daily use, they are much less aggressive than chemical peels, merely enhancing one's natural exfoliation/desquamation process, and their effect is limited to the upper layers of the stratum corneum. Cosmetic products include, but are not limited to, skin cleansers and washes; skin-care creams, lotions, and powders; facial make-ups including bases, foundations and blushes; shampoos and other hair care products that also are to be worked into or applied to the scalp; and the like. In following, the present invention also provides for a method of enhancing the appearance of skin and/or skin tone by the application on a daily or other repetitive basis of a skin care or other cosmetic product containing low levels of the two actives.

These and other features, aspects, and advantages of the present invention will become evident to those skilled in the art from reading of the present disclosure.

DESCRIPTION OF THE INVENTION

As used herein and in the appended claims, the phrase "substantially free of when used in conjunction with or in relation to meroterpenes means that the recited compound or component, if present, is present at an inconsequential level, generally less than 0.1 wt% based on the weight of the meroterpene. Similarly, the term "dermatologically-acceptable", as used herein, means that the compositions or components thereof so described are suitable for use in contact with human skin without undue toxicity, incompatibility, instability, irritation, allergic response, and the like.

Dermatological Acids

The first of the two critical ingredients of the compositions and products of the present invention is a dermatological acid. Dermatological acids are characterized as those carboxylic acids, naturally derived or synthetically produced, that cause or enhance the exfoliation/desquamation of skin cells from the epidermis and/or the removal of comedones and other debris from the skin surface, pores and follicles. The degree of exfoliation or depth of the exfoliation/desquamation and the extent of skin cleansing depends upon the concentration and strength of the dermatological acid in the compositions and products to be applied to the skin and the rate of application of the products themselves, both in terms of amount applied and frequency of application.

Dermatological fatty acids include those fatty acids having one or more carboxyl (COOH) groups, particularly at least one carboxyl end group, and from 6 to 20 carbon atoms, inclusive of those of the carboxyl group. Analogs include the lower alkyl esters thereof, e.g., the $C_1$ to $C_4$ alkyl esters, especially the ethyl esters. Dermatological fatty acids include azelaic acid, lauric acid, myristic acid, stearic acid, palmitic acid, arachidic acid, behenic acid, linoleic acid, alpha-linoleic acid, oleic acid, arachidonic acid, and the like.

Benzylic acids are generally of the formula (I):

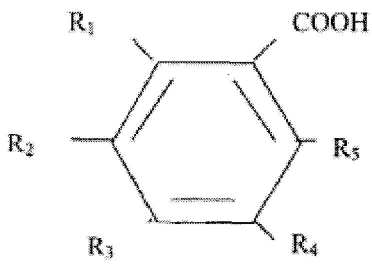

wherein $R_1$, $R_2$, $R_3$, $R_4$ and $R_5$ are each independently selected from the group consisting of H, OH, F, I Br, Cl, SH, $NH_2$, $C_{1-4}$ alkyl, $C_{1-4}$ alkoxyl, OR, COR, COOR, $CONR_2$ and $SO_3R$ wherein R is independently selected from H, $C_{1-4}$ alkyl and $C_{1-4}$ alkoxyl groups. Compounds of this formula include benzoic acid, 3-hydroxy benzoic acid, and salicylic acid.

Retinoids are compounds that are chemically related to vitamin A and generally include a cyclic group having a polyene side chain (typically having conjugated double bonds) with a polar end group. Retinoids include first generation retinoids such as retinol, tretinoin, and isotretinoin; second generation retinoids such as etretinate and acitretin; and third generation retinoids such as tazarotene, adapalene and bexarotene. Useful retinoids include adapalene, tretinoin, isotretinoin, and tazarotene.

Sugar acids include the glyceric acids (acids based on glycerol), ascorbic acid, and glucuronic acid.

Hydroxy acids are aliphatic, cyclic, heterocyclic and aromatic carboxylic acids having at least one hydroxyl group in addition to the carboxylic group or groups. The most common hydroxy acids are the alpha- and beta- hydroxy acids, i.e., those hydroxy acids where the hydroxyl group is on the carbon atom next to or once removed from, respectively, the carboxyl carbon atom. Hydroxy acids may have a single hydroxyl group or a plurality of the same as well as a single carboxyl group or a plurality thereof. Furthermore, they may be used alone or in combination with other hydroxy acids or derivatives thereof, especially the simple esters (especially the $C_{1-4}$ alkyl esters) and ammonium salts thereof. Hydroxy acids include, but are not limited to: glycolic acid, lactic acid, citric acid, malic acid, tartaric acid, mandelic acid, gluconic acid, glycolic acid + ammonium glycolate, alpha-hydroxyethanoic acid + ammonium alpha-hydroxyethanoate, alpha-hydroxyoctanoic acid, alpha-hydroxycaprylic acid, hydroxycaprylic acid, mixed fruit acid, triple fruit acid, tri-alpha hydroxy fruit acids, sugar cane extract, alpha hydroxy and botanical complex, L-alpha hydroxy acid, salicylic acid, beta hydroxybutanoic acid, tropic acid, and trethocanic acid.

Other dermatological acids include, for example, trichloroacetic acid, the keto acids such as pyruvic acid and ethyl pyruvate, and the like.

Typically, the salicylic acid is used in the amount previously employed, though as noted below, it has now been found that a synergy is oftentimes manifest between the dermatological acid and the bakuchinol such that lower amounts of the acid may be employed for the same result. Generally speaking, the acids will be employed in an amount of from 10 percent or less to about 70 percent, or slightly more. Most typically, over the counter products sold for consumer use will have around 10 percent or less. The concentration of acid in products used by trained cosmetologists in spas, salons and the like may run between 20 and 30 percent, while those used by doctors can range from 50 to 70 percent.

Meroterpene

The second critical component of the skin treatment compositions of the present invention is the meroterpene bakuchinol. Meroterpenes are terpenes having an aromatic ring and are generally of the following chemical structure (I):

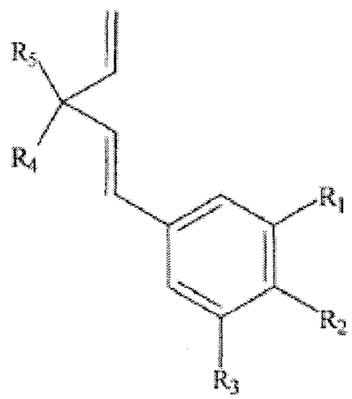

wherein $R_1$, $R_2$, and $R_3$ are each independently selected from the group consisting of H, OH, $OR_6$ or $CH_2R_6$ where $R_6$ is linear or branched $C_1$ to $C_8$ alkyl; and $R_4$ and $R_5$ are each independently a linear or branched, $C_1$ to $C_{20}$ alkyl or alkenyl group. Meroterpenes include Bakuchiol wherein $R_1$=$R_3$=H; $R_2$=OH, $R_4$=$CH_3$; $R_5$=$CH_2CH_2CH$=$C(CH_3)_2$ and Corylifolin wherein $R_1$=$R_3$=H; $R_2$=OH, $R_4$= $R_5$=$CH_3$.

Meroterpenes are typically derived from plants and plant extracts, though they have also been obtained from fungi as well as produced synthetically. Plants and plant extracts, though, remain the most common source of these compounds with Psoralea coryfolia, Psoralea grandulosa, and Otholobium pubescens (Fabaceae) being the more common of such plant sources. In the practice of the present invention, the bakuchinol may be added as an isolated or purified material of at least 60% purity w/w, preferably at least 95% pure w/w, or it may be added in the form of a purified plant extract containing 1 to about 70% or more by weight meroterpene based on the total weight of the extract. As used herein the phrases "purified plant extract" and "purified extract" means that the extracts are purified to remove coumarins and other deleterious constituents, as discussed below, without specific isolation and recovery of the specific meroterpene and/or multiple fractions are combined or the collection time and temperatures for a given fraction are longer than would be used to isolate a specific meroterpene. The meroterpene must be free or substantially free of coumarins, especially furocoumarins like psoralene and iso-psoralene, and other like compounds that are skin sensitizers and/or enhance the detrimental effect of UV exposure. Because such materials are also found in the same plants and extracts, they are typically present in commercial grade meroterpenes and meroterpene extracts. For example, psoralens and iso-psoralens typically comprise 0.1 to 2% of the dry weight of the plant and seed source materials and from about 1.0 to 20% by weight of the crude extracts thereof in organic solvents such as ethanol.

The preferred meroterpene for use in the practice of the present invention is Bakuchiol. Bakuchiol is a known bio-active material and has been used as an antitumor agent, an antimicrobial agent, an anti-inflammatory agent, a skin whitening agent, etc. It, in combination with pyridine aldehyde, has also been used in the treatment of pimples, acne, blackheads, herpes, and other skin disorders. However, its use is limited or at least tempered by the presence of high levels of psoralene and other furocoumarins. Although psoralene is a strong bio-active agent, - it is used in combination with UVA for the treatment of psoriasis and eczema - it greatly enhances the sensitivity of skin to the effects of UV exposure, thereby, significantly increasing the potential for sunburn. Consequently, the use of Bakuchiol and other meroterpenes, particularly those derived from plant sources, as a treatment has been limited to circumstances were sun exposure is not of concern or where precautions are taken to avoid sun exposure following treatment.

Recent developments, however, have been made in meroterpene production and recovery enabling one to prepare meroterpenes that are free or substantially feel free of coumarins, especially if furocoumarins like psoralene and isopsoralene. For example, Indian patent publication no. 00570/KOL/2005, (filed in June 29, 2005 and published on January 13, 2006) describes a method of purifying Bakuchiol from the extract of Psoralea corylifolia seeds. The method involves extraction of the plant material (powdered seeds) with a non-polar solvent like hexane heptane. The extract solution is then treated with an alkali solution such as an alkali metal carbonate, bicarbonate or hydroxide to provide a 3-layered volume liquid, an organic layer, an emulsion layer and an aqueous layer. The organic layer is washed with water and dilute HCl and concentrated to a viscous mass. Concurrently, the emulsion layer is dissolved in a polar solvent like ethyl acetate and separated to remove the so-formed aqueous layer. The aforementioned viscous mass is then mixed with the ethyl acetate solution and concentrated to remove ethyl acetate and traces of the non-polar solvent. The concentrated mass is then subjected to high vacuum distillation, generally 1 mm to 0.1 mm at 139°C to 175° C. That fraction collected between the oil bath temperature of 190-270° C and vapor temperature range of 140-180° C is found to contain pure Bakuchiol, free or substantially free of psoralene and isopsoralene as well as other known constituents of such plant extracts such bavachicin, bavachin, angelicin, isobavachalcone, bakuchcin, and the like.

A similar method for the preparation of Bakuchiol that is free or substantially free of impurities, particularly furocoumarin impurities, is described in Jia et. al. - US 2006/0251 749. Jia et. al. describes a method wherein the plant source materials are subjected to an extraction and the extract solutions are then subject to hydrolysis with a basic solution such as aqueous sodium hydroxide. The resultant product is then purified by one of column chromotography, extraction followed by crystallization, solvent partition, recrystallization, and combinations of the foregoing. Crude extracts purified in this way are said to be essentially free of furocoumarins such as psoralene and isopsoralene.

Other publications or patents that describe isolation or synthesis of meroterpenes include:

CN Backhouse, CL Delporte, RE Negrete, S Erazo, A Zuniga, A Pinto, BK Cassels, J Ethnopharmacology, 78(1):27-31, 2001.
H Haraguchi, J Inouye, Y Tamara, K Mizutani, Planta Medica, 66(6):569-571, 2000.
JM Krenisky, J Luo, MJ Reed, JR Carney, Biol Pharm Bull, 22(10):1137-1140, 1999.
H Katsura, R Tsukiyama, A Suzuki, M Kobayashi, Antimicrobial Agents and Chemotherapy, 45(11):3009-3013, 2001.
S Adhikari, R Joshi, BS Patro, TK Ghanty, GJ Chintalwar, A Sharma, S Chattopadhaya, T Mukherjee, Chem Res Toxicol, 16:1062-1069, 2003.

JB Perales, NF Makino, DL Van Vranken, J Org Chem, 67:6711-6717, 2002,

all of which are incorporated herein by reference in their entirety. These purified meroterpenes, especially the purified bakuchiol, may be obtained from Sytheon Ltd., of Lincoln Park, NJ, USA and Unigen Pharmaceuticals, Inc. of Lacey, WA, USA.

The presence of the meroterpene in the compositions and products of the present invention is found to reduce certain of the adverse consequences and/or side effects of the dermatological acid. Specifically, it is found that the presence of the meroterpene reduces the severity and/or longevity of the manifestation of skin irritation, reddening and inflammation associated with the use of dermatological acids. This is particularly noticeable with those skin treatment compositions and cosmetic products wherein weak or weaker acids and/or lower concentrations of such acids are present, for example, general cosmetic products, acne and other skin disorder treatments and over the counter treatments, and in consumer and cosmetologist applied chemical peels. However, the effect is less noticeable in those skin treatment compositions and cosmetic products where stronger acids and/or significantly higher concentrations are used. For example, little effect is noted on skin irritation, reddening and the like with the stronger, professionally applied chemical peels due to the very nature of that type of skin peel.

Nevertheless, the presence of the meroterpene has been found to have a secondary benefit, namely a reduction in skin sensitivity or at least the manifestation of skin sensitivity to UV light resulting from the use of dermatological acids. Specifically, since dermatological acids result in the removal of skin layers, especially the uppermost layers of dead skin that serve, in part, as barriers to UV light, the underlying and newly exposed skin is either more sensitive or receives a larger dose of UV energy resulting in enhanced erythema and other adverse consequences of exposure, especially overexposure to UV light. It is now found that the presence of the meroterpene counteracts that consequence, reducing the degree of erythema for a given exposure and/or shortening the recovery period over which the erythema is lost. Indeed, with short duration UV exposures, the presence of the meroterpene may result non-appearance, at least to the human eye, of any erythema.

Surprisingly, it has also been found that the meroterpene oftentimes results in an enhancement in the effectiveness of or a synergy with the dermatological acid. For example, when combined with dermatological acids for treatment of acne and other skin disorders, especially bacterial related disorders, the rate of cure and degree of cure is enhanced. Also, the degree and severity of scarring and other effects of acne and such disorders is less. Additionally, and in following, when used in general skin care and cosmetic products, the presence of the meroterpene is found to provide a better overall skin tone and appearance as compared to similar formulations without the meroterpene. Thus, while originally intended to counteract some of the adverse consequences and side effects of dermatological acids, it has been found that the meroterpenes actually enhance their effectiveness and/or provide an additional benefit on its own that reflects a synergy or at least seems to reflect a synergy.

The meroterpene is present in the skin treatment compositions and cosmetic products in an effective amount, that is, in an amount that reduces erythema from UV exposure as compared to the same formulation without the meroterpene. Reference to effective amount was made with respect to the manifestation of erythema following UV exposure, but could just as appropriately have been made in reference to reddening and irritancy of skin following chemical peel. In general, the amount of meroterpene to be used is not so dependent upon the application as is the selection and amount of acid. Nevertheless, it is to be recognized that one is more likely to use higher amounts of the meroterpene with stronger acids and/or higher concentrations of acids. Typically, the skin treatment compositions and cosmetic products according to the present invention will contain from about 0.1 to about 10, preferably from about 0.5 to about 5, weight percent of the meroterpene based on the total weight of the skin treatment composition or cosmetic product. Higher levels could be used but are not thought to be necessary. When the meroterpene is added as a purified plant extract or as a purified material that also contains other components, the weight percent is based on the meroterpene content itself.

Dermatologically Acceptable Carrier

The second critical component of the skin treatment compositions and cosmetic products of the present invention is the dermatologically acceptable carrier. The carrier is that material or combination of materials that is used to essentially carry or deliver the actives to the skin. The carrier can be in a wide variety of forms including, for example, liquids, lotions, creams, gels, foams, emulsions, dispersions, sprays, liposomes, coacervates, ointments, etc. For example, the carrier may be an aqueous-based solution or cleanser; an alcohol-based solution or gel; an ointment based on fats, waxes, animal and vegetable oils, and solid and liquid hydrocarbons; or an emulsion carrier, including, but not limited to, oil-in-water, water-in-oil, water-in-oil-in-water, and oil-in-water-in-silicone emulsions, etc. That carrier can also be, for example, formulated as alcohol or water based cleansers, toilet bars, liquid soaps, shampoos, bath gels, hair conditioners, hair tonics, pastes or mousses. The carrier will generally comprise from about 30% to about 99.99%, preferably from about 50% to about 99.9%, more preferably from about 80% to about 99%, most preferably from about 85% to about 95% of

the skin treatment composition or cosmetic product based on the combined weight of the actives and the carrier.

Generally speaking, any known carrier or base composition employed in traditional cosmetic and/or dermatological applications/compositions as well as pharmacological skin treatment compositions, particularly topically applied pharmacological skin treatment compositions, may be used in the practice of the present invention. Suitable carriers and carrier compositions are described at length in, for example, Gonzalez et. al. - US 7,186,404; Aust et. al. - US 7,175,834; Roseaver et. al.- US 7,172,754; Simoulidis et. al. - US 7,175,835; Mongiat et. al. - US 7,101,536; Maniscalco - US 7,078,022; Forestier et. al. US 5,175,340, US 5,567,418, US 5,538,716, and US 5,951,968; Deflandre et. al. - US 5,670,140; Chaudhuri - US 7,150,876, US 6,831,191, US 6,602,515, US 7,166,273, US 6,936,735, US 6,831,191, and US 6,699,463; Chaudhuri et. al. - US 6,165,450 and US 7,150,876; Bonda et. al. US 6,962,692; and Wang et. al. US 5,830,441, all of which are incorporated herein by reference in their entirety.

Obviously, the selection of the carrier and type of carrier depends upon a number of factors including the intended use and objective of the inventive compositions and products and the compatibility of the two active components with the components of the carrier. For example, a chemical peel to be administered by a trained medical professional may merely require the actives and a suitable solvent for the same. Though, in this instance, one would preferably select a solvent that is not an irritant, particularly not to the underlying skin layers that become exposed as the dermatological acid does its work. On the other hand, it may be desirable to include an anesthetic ingredient that helps numb the pain of the aggressive chemical peel and/or a topical antibiotic component to protect the treated skin from subsequent infection in its weakened state. The carrier for a therapeutic treatment for acne, on the other hand, may include a whole host of other constituents including a water soluble hydrophilic polymer to help bind the actives to the site of application, moisturizers or humectants to help maintain moisture in the skin to avoid too much peeling and/or cracking and drying of the skin; colorants and cosmetic base to mask over or cover the afflicted area of the skin. Finally, as to cosmetics, as know to those skilled in the art, such compositions will likewise include a whole host of constituents for various purposes including moisturizers, colorants, light diffusers, softening agents, and the like. Those skilled in the art will readily recognize and appreciate what carriers may be employed in light of the intended form and/or delivery method for the inventive skin treatment compositions and cosmetic products.

It is also to be noted that the pH of the formulation plays an important factor in the availability of the acid for its intended function and in the stability of the inventive compositions and products. For example, for chemical peels and therapeutic treatments, especially in the treatment of acne, a low pH is necessary in order to suppress ionization and enhance the penetration of the acid into the stratum corneum. Preferably, the pH range is from about 2.5 to about 5.5, more preferably from about 3 to about 4. However, while stability is still an issue, for daily topical applications, especially for daily cosmetic products, the pH is preferably slightly higher, preferably from about 4.0 and about 6.5, most preferably from about 4.5 to about 5.5.

A wide variety of acids, bases, and buffers can be utilized to adjust and/or maintain the pH of the compositions useful in the present invention. Examples of materials useful for adjusting and/or maintaining the pH include, without limitation, ammonia, sodium carbonate, sodium hydroxide, triethanolamine, hydrochloric acid, phosphoric acid, sodium hydrogen phosphate, sodium dihydrogen phosphate, citric acid, and the like.

Though a carrier by itself is sufficient, the inventive skin treatment compositions and cosmetic products of the present invention may, and preferably will, contain various other components typically associated with therapeutic and non-therapeutic skin care products and cosmetics. For example, various skin care agents including, but not limited to, conventional skin care excipients, actives, and photoprotective agents may be present. Such additives include, but are not limited to sunscreens, antioxidants, vitamins, anti-inflammatory agents, moisturizers, tactile modifies, emollients, preservatives, anti-inflammatory agents, penetrants, humectants, and the like, and mixtures thereof, in their conventional amounts. Exemplary agents and additive materials are described briefly below as well as in the aforementioned patents, especially Maniscalco - US 7,078,022. Each of these additives is employed in their conventional amounts, as is known to those skilled in the art.

Suitable antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, tocopherols (e.g., tocopherol acetate), tocotrienols, curcurmin and its derivatives and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, *Phyllanthus emblica* and propolis. Other examples of antioxidants may be found on pages 1612-1613 of the ICI Handbook as well as in Ghosal - US 6,124,268, both of which are incorporated herein by reference in their entirety.

The skin treatment compositions and cosmetic products of the present invention may also include one or more vitamins and/or their derivatives. Vitamins and vitamin derivatives include, for example, vitamin A, vitamin A propionate, vitamin A palmitate, vitamin A acetate, retinol, vitamin B, thiamine chloride hydrochloride (vitamin B.sub.1), riboflavin

(vitamin B.sub.2), nicotinamide, vitamin C and derivatives (for example ascorbyl palmitate, ascorbyl glucoside, and ascorbyl acetate), vitamin D, ergocalciferol (vitamin D.sub.2), vitamin E, DL-α-tocopherol, tocopherol E acetate, tocopherol hydrogensuccinate, vitamin K.sub.1, esculin (vitamin P active ingredient), thiamine (vitamin $B_1$), nicotinic acid (niacin), niacinamide, pyridoxine, pyridoxal, pyridoxamine, (vitamin $B_6$), pantothenic acid, biotin, folic acid and cobalamine (vitamin $B_{12}$). Preferred vitamins are, for example, vitamin A palmitate, vitamin C and derivatives thereof, DL-α-tocopherol, tocopherol acetate, nicotinic acid, pantothenic acid and biotin. Vitamin E, which is often added to cosmetic and personal care products is also preferably stabilized by a suitable stabilizer according to the invention.

Non-vitamin antioxidants are also suitable including, but are not limited to, BHT (butylated hydroxy toluene), L-ergothioneine (available as Thiotane™); tetrahydrocurcumin, carnosine, diethylhexyl syringylidene malonate (available as Oxynex® ST or Oxynex® ST Liquid from EMD Chemicals/Merck, Germany.), ubiquinone (co-enzyme Q10), Idebenone and combinations thereof.

Suitable emollients include those agents known for softening the skin which may be selected from hydrocarbons, fatty acids, fatty alcohols and esters. Petrolatum is a common hydrocarbon type of emollient conditioning agent. Other hydrocarbons that may be employed include alkyl benzoate, mineral oil, polyolefins such as polydecene, and paraffins, such as isohexadecane. Fatty acids and alcohols typically have from about 10 to 30 carbon atoms. Illustrative are myristic, isostearic, hydroxystearic, oleic, linoleic, ricinoleic, behenic and eruicic acids and alcohols. Oily ester emollients may be those selected from one or more of the following, triglyceride esters, acetoglyceride esters, ethoxylated glycerides, alkyl esters of fatty acids, ether esters, polyhydric alcohol esters and wax esters. Additional emollients or hydrophobic agents include $C_{12}$ to $C_{15}$ alkyl benzoate, dioctyladipate, octyl stearate, octyldodecanol, hexyl laurate, octyldodecyl neopentanoate, cyclomethicone, dicapryl ether, dimethicone, phenyl trimethicone, isopropyl myristate, capriylic/capric triglycerides, propylene glycol dicaprylate/dicaprate and decyl oleate, cyclomethicones and other silicone derivatives.

Suitable humectants include various polyhydric alcohols, especially polyalkylene glycols and, more preferably, alkylene polyols and their derivatives. Exemplary humectants include propylene glycol, dipropylene glycol, polypropylene glycol, polyethylene glycol, sorbitol, 2-pyrrolidone-5-carboxylate, hydroxypropyl sorbitol, hexylene glycol, ethoxydiglycol 1,3-butylene glycol, 1,2,6-hexanetriol, glycerin, ethoxylated glycerin, propoxylated glycerin, compatible solutes, such as ectoin, hydroxyectoin, taurines, carnitine, acetyl carnitine and mixtures thereof. When employed in effective amounts, generally from 1 to 30%, preferably from 2 to 20%, by weight of the skin lightening/even toning composition, these additives serve as skin moisturizers as well as reduce scaling and stimulate the removal of built-up scale from the skin.

With the more aggressive compositions, especially those containing moderate to stronger dermatological acids and/or dermatological acids in higher concentrations, most especially chemical peels and pharmaceutical grade skin treatments, it may also be desirable to include one or more anti-inflammatory agent. Examples of anti-inflammatory ingredients include, but are not limited to, bisabolol, curcurmin and its derivatives, retinoids, flavonoids, terpenes and other polyphenolics etc. These and other anti-inflammatory agents are disclosed in Gupta et. al. - US 2005/0048008A1, which is incorporated herein by reference in its entirety. Compositions containing steroidal anti-inflammatory, non-steroidal anti-inflammatory, as well as "natural" anti-inflammatory, such as extract of the plant Aloe vera, are also included in the present invention and have been disclosed for such use. See e.g., U.S. Pat. No. 4,185,100, Rovee, issued Jan. 22, 1980 (hydrocortisone, dexamethasone, naproxen, ketoprofen, ibuprofen); U.S. Pat. No. 4,338,293, Holick, issued Jul. 6, 1982 (steroidal anti-inflammatories); Law, et al., Br. J. Pharmac., 59(4), 591-597 (1977) (ibuprofen); Kaidbey, J. Invest. Dermatoloy, 66, 153-156 (1976) (indomethacin); and Gruber, et al., Clinical Pharm. and Therapeut., 13(1), 109-113 (1971) (aspirin, fenoprofen).

Furthermore, those skin treatment compositions and, especially, the cosmetic products of the present invention that are intended to stay on the skin during the daylight hours may contain and preferably do contain one or more sunscreen actives. Sunscreen actives are of two types, inorganic actives that work by reflecting the UV light and organic actives that work, predominately, by absorbing UV energy. The amount of the sunscreen active to be incorporated into the sunscreen formulations is that which is conventional in the art. Typically, the amount is dependent upon, among other factors, the delivery means, e.g., is it applied as a spray or lotion; the stability of the active; the efficacy of the selected sunblock active itself; and the application rate, as well as the particular SPF desired. From the commercial perspective, another factor influencing the level of such sunscreen actives in the sunscreen formulations is the regulatory limitations on their use. In the United States, for example, strict controls are placed upon the maximum level at which approved sunscreen actives may be present. Regulatory controls may also dictate which sunscreen actives may be used in which countries.

Suitable organic sunscreen actives include, for example, butyl methoxydibenzoylmethane (avobenzone), benzophenone-8, dioxybenzone, homosalate, octylsalate, menthyl anthranilate, octocrylene, ethyhexyl methoxycinnamate (Octinoxate), oxybenzone, ethylhexyl salicylate (Octisalate), benzophenone-3, ethylhexyl dimethyl PABA (Padimate O), glyceryl PABA, phenylbenzimidazole sulfonic acid, sulfisobezone, trolamine salicylate, 4-methylbenzylidene camphor, bisoctriazole, bemotrizinol, ecamsule, drometrizole trisiloxane, disodium phenyl dibenzimidazole tetrasulfonate, diethylamine hydroxybenzoyl hexyl bezoate, octyl triazone, hexyl benzoate, benzophenone-4, ethyhexyl triazone, diethylhexyl butamido triazone, bisimidazylate, polysilicone-15, etc.

Inorganic sunscreens include, but are not limited to, microfine surface treated titanium dioxide and microfine untreated and surface treated zinc oxide. The titanium dioxide in the sunscreen compositions preferably has a mean primary particle size of between 5 and 150 nm, preferably between 10 and 100 nm. Titanium oxide may have an anatase, rutile, or amorphous structure. The zinc oxide in the sunscreen compositions preferably has a mean primary particle size of between 5 nm and 150 nm, preferably between 10 nm and 100 nm.

Examples of suitable hydrophobically modified titanium dioxide compositions include but are not limited to: UV Titans® X161, M160, M262 (surface treated with stearic acid and alumina) (Kemira); Eusolex ® T-2000 (surface treated with alumina and simethicone) (Merck KGaA); T-Cote® (surface treated with dimethicone) (BASF); Mirasun® TiW60 (surface treated with silica and alumina) (Rhodia); Tayaca MT100T (surface treated with aluminum stearate) (Tayaca); Tayaca MT-100SA (surface treated with silica and alumina) (Tayaca); Tayaca MT-500SA (surface treated with silica and alumina) (Tayaca); Tioveil® EUT, FIN, FLO, FPT, GCM, GPT, IPM, MOTG, OP, TG, TGOP (surface treated with silica and alumina, 40% dispersion in a range of cosmetic vehicle) (ICI); Eusolex® T-45D (surface treated with alumina and simethicone, 45% dispersion in isononoylnonaoate) (Merck KGaA); and Eusolex® T-Aqua (surface treated with aluminum hydroxide, 25% dispersion in water) (Merck KGaA).

Examples of suitable untreated and hydrophobically modified zinc oxide include but are not limited to: Z-Cote® (uncoated microfine zinc oxide) (BASF); Z-Cote® HP-1 (surface treated with dimethicone) (BASF); Sachtotec® LA 10 (surface treated with lauric acid) (Sachtleben); Sachtotec® (uncoated microfine zinc oxide) (Sachtleben); Spectraveil® FIN, IPM, MOTG, OP, TG, TGOP (uncoated, 60% dispersion in a range of cosmetic vehicle) (ICI); Z-sperse® TN (untreated, dispersion in C12-15 alkyl benzoate) (Collaborative); Z-sperse® TN (untreated, dispersion in octydodecyl neopentanoate) (Collaborative).

Most preferably, a combination of such sunscreen actives will be employed. In this respect, it is well known that certain sunscreen actives have better stability, hence longevity, than others; while others have better absorptive capabilities, whether in reference to selectivity for certain UV energy of certain wavelength(s) or cumulative absorptive capabilities. If needed, suitable photostabilizer, for examples, diethylhexyl bezylidene malonates (Oxynex® ST or Oxynex® ST Liquid marketed by EMD/Merck, Germany), 4-methylbenzylidene camphor, butyloctyl salicylate, diethylhexyl 2,6-naphthalate (Corapan® TQ, marketed by Symrise) etc. can also be included to stabilize unstable sunscreen actives. Additionally, synergistic agents may be used in combination with one or more sunscreen compositions including for example bakuchiol. Such synergistic combinations are disclosed in, e.g., the patent application of Ratan Chaudhuri for "Sunscreen Compositions and Methods" filed on May 14, 2007 as Application Serial No. 11/803188, which is incorporated herein by reference in its entirety. Hence, by using combinations of such UV sunscreen actives, one is able to provide greater prevention of sun-induced hyperpigmentation. Suitable combinations of sunscreen actives are well known in the art and within the skill of a typical artisan in the field.

On the other hand, chemical peels according to the present invention as well as therapeutic skin treatment compositions, such as for the treatment of acne, will further include one or more skin penetrants to enhance and expedite the penetration of the dermatological acid through the skin layers to effectuate a deeper effect. Skin penetrants are additives that, when applied to the skin, have a direct effect on the permeability of the skin barrier: increasing the speed with which and/or the amount by which certain other compounds are able to penetrate into the skin layers. Exemplary organic penetration enhancers include dimethyl sulfoxide; isopropyl myristate; decyl, undecyl or dodecyl alcohol; propylene glycol; polyethylene glycol; $C_{9-11}$, $C_{12-13}$ or $C_{12-15}$ fatty alcohols; azone; alkyl pyrrolidones; diethoxy glycol (Transcutol); lecithin; etc. Surfactants can also be used as penetration enhancers.

Other optional adjunct ingredients for the skin treatment compositions and cosmetic products of the present invention include preservatives, waterproofing agents, fragrances, anti-foam agents, plant extracts (Aloe vera, witch hazel, cucumber, etc), opacifiers, stabilizers, skin conditioning agents colorants, and the like, each in amounts effective to accomplish their respective functions.

The skin care treatment compositions and cosmetic products of the present invention may be used to effect various objectives. In this light, these compositions may be formulated to effectuate a chemical peel; to treat an adverse skin condition, especially acne or rosacea; or to generally improve the overall appearance and/or tone of one's skin. In each method, the appropriate formulation is applied to the surface of the skin to be treated. The material is either allowed to remain, as with a cosmetic or pharmaceutical application, or is removed following a given period of time, as with a chemical peel. The amount of the topical composition to be applied to the skin will again depend upon the specific composition or product and its objective. Generally speaking, each composition or product will be applied in an amount and manner traditionally employed for such comparable conventional products that are free of the meroterpenes. For most applications, though, the quantity of the inventive compositions and products to be applied per application are, in mg composition/$cm^2$ skin, from about 0.1 mg/ $cm^2$ to about 10 mg/ $cm^2$. A particularly useful application amount is from about 1 mg/$cm^2$ to about 2 mg/$cm^2$.

The frequency and period of application for the skin treatment compositions and cosmetic products of the present invention will also vary widely depending upon the nature of the composition or product, including its form, concentration, intended function, etc. In essence, each is applied in an amount and for a sufficient period of time and

with such frequency as is necessary to achieve the objective of the dermatological acid component. In the case of a chemical peel, the compositions would be applied one time, for a brief period of time and in an amount to sufficient to wet the surface of the skin. As noted above, chemical peels are to be left on the skin for only a brief period of time depending upon the aggressiveness of the same. In a professional or medical setting, due to the extremely aggressive nature of the chemical peel compositions employed, the compositions is applied for only a few minutes, perhaps 3 to 10 minutes. For less aggressive compositions, those that are typically applied in a salon or spa or that are used for home treatment, the compositions may be left on for 10 minutes or longer, perhaps up to 30 minutes or more. Generally, it should not be left on for more than one hour. Thereafter it is washed off or first neutralized and then washed off. Chemical peels may be repeated but on an infrequent basis. Professionally applied peels may take weeks if not months to recover: thus once or maybe twice a year applications may be appropriate. Spa, salon and home applied peels are less aggressive and can be applied on a more frequent basis: generally every few months or so.

In the case of an anti-acne treatment, rosacea treatment, or another similar skin disorder treatment, the compositions would typically be applied once or twice daily in an amount sufficient to wet or cover the afflicted skin and left in place. These compositions would typically be re-applied for so long as necessary, generally until the disorder is no longer present: though its application could be continued thereafter for a month, a year, or more, for prophylactic or preventative purposes. Of course, it is to be appreciated that in the treatment of certain skin disorders it may be appropriate or desirable to employ an aggressive and/or a higher concentration of dermatological acid to mimic a chemical peel. In such instances, the duration and frequency of application would be similar to those mentioned above for chemical peels or more intermediate those for peels and those for most skin treatment compositions.

Cosmetic products and applications, i.e., those intended to enhance skin tone and appearance, are to be applied to the skin on a chronic basis. Typically, such applications would be on the order of about one or more times each day over extended periods - weeks, months, even years - to reverse and/or delay the effect of aging and/or in an effort to maintain a more youthful skin tone and appearance. While a maximum benefit or noticeable change in skin appearance may be achieved after several months or years of use, one may continue to apply the cosmetic product daily or less frequently, perhaps once or twice a week, in order to maintain that skin condition or at least delay or lessen the impact of aging. These products are typically of the type that are intended to be "left on" once applied, generally for an hour or more, preferably several hours, even 12 or more hours.

Delivery Methods for the Compositions

The skin treatment compositions and cosmetic products according to the present invention can be applied to any skin surface for its intended function. While most commonly applied to the external surface of the face and/or certain facial features including face, lips, under eye area, eyelids, eyebrows, ears, and cheeks; they are equally applicable to the scalp, neck, shoulders, arms, torso, feet, hands, fingers, toes, etc. The compositions and products according to the present invention are preferably applied in the form of a lotion, cream, gel, foam, ointment, paste, emulsion, dispersion, liposome, coacervate, spray, conditioner, tonic, cosmetic (including foundation, base and other make-up), lipstick, after-shave, skin cleanser, moisturizer, or the like. Alternatively, the compositions and products according to the present invention may be in the form of a substrate-based product wherein the inventive composition is impregnated into or onto a substrate, most typically a woven and/or non-woven wipe, pad, patch, tissue, mask, and the like.

The compositions and products according to the present invention may be delivered directly from the package or container to the skin to be treated, transferred to the users hand or fingers for application, or to another substrate for application including, for example, pads, cotton balls, wipes, masks, applicators and the like. In the case of direct application, the composition may be squeezed from a tube or sprayed from a spray container. Alternatively, there are a number of applicator devices that carry or store the composition and are configured to deliver them as well. For example, the compositions can be incorporated into and delivered from a soft-tipped or flexible dispensing device. These devices are useful for the controlled delivery of the compositions to the skin surface and have the advantage that the treatment composition itself never need be directly handled by the user. Non-limiting examples of these devices comprise a fluid container including a mouth, an applicator, means for holding the applicator in the mouth of the container, and a normally closed pressure-responsive valve for permitting the flow of fluid from the container to the applicator upon the application of pressure to the valve. The valve can include a diaphragm formed from an elastically fluid impermeable material with a plurality of non-intersecting accurate slits therein, where each slit has a base which is intersected by at least one other slit, and where each slit is out of intersecting relation with its own base, and wherein there is a means for disposing the valve in the container inside of the applicator. Examples of these applicator devices are described in U.S. Pat. No. 4,693,623 (Schwartzman), U.S. Pat. No. 4,620,648 (Schwartzman), U.S. Pat. No. 3,669,323 (Harker et al.), U.S. Pat. No. 3,418,055 (Schwartzman) and U.S. Pat. No. 3,410,645 (Schwartzman), all of which are incorporated herein by reference in their entirety.

As noted above, the inventive compositions can be impregnated into a substrate-based delivery means. For example, in order to ensure a continuous exposure of the skin to at least a minimum level of the dermatological acid

and meroterpene, the composition may be impregnated into a patch that is then applied to the skin. Such an approach is particularly useful for acne-affected skin areas and other skin disorders needing more intensive treatment. The patch can be occlusive, semi-occlusive or non-occlusive and can be adhesive or non-adhesive. The meroterpenes and the additional skin acne care active (or actives) composition can be contained within the patch or be applied to the skin prior to application of the patch. The patch is preferably left on the skin for a period of at least about 5 minutes to as long as 1 hour or more preferably at night as a form of night therapy.

Another substrate-based delivery means is the cleansing pad. Preferably these pads comprise from about 50% to about 75% by weight of one or more layers of non-woven fabric material and from about 20% to about 75% by weight (on dry solids basis) of a water soluble polymeric resin. Examples of pads are described in U.S. Pat. No. 4,891,228, (Thaman et al.) and U.S. Pat. No. 4,891,227 (Thaman et al.), both of which are incorporated by reference herein in their entirety.

Continual, preferably daily, use of the skin treatment compositions of the present invention, regardless of whether one anticipates UV exposure or not, provide a number of additional benefits to ones skin. For example, the continual use of these skin treatment compositions will delay the appearance of fine lines, enhance extra-cellular matrix cohesion, reduce the appearance of spider veins, improving skin firmness and elasticity: skin effects that are not only a result of exposure to the sun but also the natural aging process. In essence, the long-term benefits of the continual use of the skin treatment compositions of the present invention include the lessening or delayed manifestation, possibly even the prevention or repair, of skin damage and will manifest itself in an overall improved skin quality as compared to skin on which meroterpene-free skin treatment composition had been applied on an on-going basis. For example, the long-term use of the inventive skin treatment compositions may help with thickening the keratinous tissue (i.e., building the epidermis and/or dermis layers of the skin), thereby preventing and/or retarding atrophy of human skin; preventing and/or retarding the appearance of spider veins and/or red blotchiness on human skin; preventing and/or retarding the appearance of dark circles under the eye; preventing and/or retarding shallowness and/or sagging of human skin; soften and/or smooth lips; preventing and/or relieving itch of human skin, regulating skin texture (e.g. wrinkles and fine lines), improving skin color (e.g. redness, freckles); and the like.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius; and, unless otherwise indicated, all parts and percentages are by weight.

## EXAMPLES

Having described the invention in general terms, Applicants now turn attention to the following examples in which specific formulations and applications thereof are evaluated. In the foregoing and in the following examples, unless otherwise indicated, all temperatures are set forth in degrees Celsius and all parts and percentages are by weight.

### Bakuchiol and Corylifolin

Purified Bakuchiol and Corylifolin for use in the following examples were obtained by the method described by RK Tikare and P Pujari (Indian patent application 00570/KOL/2005; publication date January 13, 2006). Specifically, seeds of *Psoralea Corylifolia* were powdered and subjected to extraction using a non-polar solvent, hexane, in a ratio of 3 ml solvent for each gram of powdered seed. The mix was stirred with heating at 60° C for 4 hrs and then filtered to separate the extract solution. The extraction was repeated three more times, for a total of four extractions on each sample of the powdered seed material, to increase the yield. Extract solution collected from all four batches was combined and the total volume reduced by distilling off excess solvent until the remaining volume was about one-half the original volume. The concentrated solution was then washed with 7.5 L of an alkali solution (5 % NaOH) twice. The alkali treatment produced three layers: an organic layer, an emulsion layer and an aqueous layer. The organic layer was washed with an equal volume of water and dilute HCl and subsequently concentrated to produce a viscous mass. The emulsion layer from the alkali washing was dissolved in a polar solvent, ethyl acetate, to isolate and remove any water that may have been present in the original emulsion layer. The remaining ethyl acetate solution was then combined with the organic layer viscous mass, produced above, and the mixture concentrated by distillation to remove ethyl acetate and any remaining hexane. The concentrated mass was then subjected to high vacuum distillation and various fractions collected. Those fractions collected between oil bath temperatures of 190°C and 270°C and vapor temperature of from 140°C to 180°C were found to contain pure Bakuchiol with less than 0.05% by weight psoralene and isopsoralene. Those fractions collected between oil bath temperatures of 140 °C and 190°C and vapor temperature of from 90°C to 150°C were subjected to column chromatographic purification to obtain pure Corylifolin with less than 0.1% psoralene. Purity was determined by HPLC analysis using a sample concentration of ≈ 0.5 mg/ml in acetonitrile , using a mobile phase composition of acetonitrile and water (70/30) with a flow rate of 1.0 ml/min., and a UV detector set at $\lambda_{max}$261 nm.

**Example 1:** Reduction of UV-induced Erythema

Erythema, the most familiar manifestation of UV radiation exposure, occurs in a biphasic manner. UV-A mediates the early part of this reaction, known as immediate pigment darkening (IPD) and lasts for about half-hour. Delayed erythema, a function primarily of UV-B dosages, begins 2-8 hours after exposure and reaches a maximum in 24-36 hours, with erythema, pruritius, and pain in the sun-exposed areas.

Microscopically, changes are detectable as early as 30 minutes after UV radiation exposure. Epidermal changes include intracellular edema, vacuolization and swelling of melanocytes, and the development of characteristic sunburn cells. In the dermis, UV radiation initially leads to interstitial edema and endothelial cell swelling. Later, there is perivenular edema, degranulation, and loss of mast cells, a decrease in the number of Langerhans cells, neutrophil infiltration, and erythrocyte extravasations. One of the primary responses to UV exposure is with respect to glutathione, a key endogenous antioxidant associated with cell protection. Specifically, UV-A exposure induces immediate loss of reduced glutathione (GSH) in both melanocytes and keratinocytes; whereas, UV-B exposure causes instant reduction of glutathione and plasma membrane instability.

In order to evaluate the anti-erythematic properties of the skin treatment compositions of the present invention two sample lotions were applied to subjects who were then subjected to UV exposure. Both lotions contained the hydroxy acid; however, only one contained the meroterpene active. Additional water was added to that formulation free of meroterpene in order ensure that the relative concentrations of all other ingredients were the same. The general formulation of the lotions evaluated were as set forth in Table 1.

The lotion was prepared by combining the Phase 1 ingredients, and then dispersing the Phase A-2 ingredient in the combined Phase A-1 composition while stirring and heating to a temperature of 75°C. Concurrently, the ingredients of Phase B were combined and heated to 75°C. Phase B was then added to Phase A with good mixing. Thereafter, the combined Phase A and B were homogenized at moderate speed, while adding Phases C and D. The complete mixture was allowed to cool to room temperature with constant propeller agitation until a homogeneous mixture was attained. The resultant mixture was found to have a pH of 6.20 and viscosity of 20,000 mPas (Brookfield RVT, Spindle C, 10 rpm) at 25°C.

**TABLE 1**

| **Ingredient** | **Trade Name / Supplier** | **wt%** |
|---|---|---|
| **Phase A-1** | | |
| Water | Water(demineralized) | 78.70/77.70 |
| Disodium EDTA | Versene Na/Dow | 0.10 |
| Glycolic acid | | 2.00 |
| Glycerine | Emery 916/Cognis | 3.00 |
| **Phase A-2** | | |
| Xanthan Gum | Vanzan NF/Vanderbilt | 0.20 |
| **Phase B** | | |
| Caprylic /Capric Triglyceride | Myritol 318/Cognis | 6.00 |
| Squalane | Fitoderm/Centerchem | 1.00 |
| Cetyl Esters | Crodamol SS/Croda | 1.00 |
| Cetyl Alcohol | Crodacol C-70/Croda | 1.00 |
| Dimethicone | DC 200, 50 cst/Dow Corning | 2.00 |
| Glyceryl Stearate, PEG-100 Stearate | Arlacel 165/Uniquema | 3.50 |
| ***Bakuchiol*** | ***Sytenol™ A / Sytheon*** | 0.0/1.0 |
| **Phase C** | | |
| Hydroxyethyl Acrylate/Sodium Acryloyldimethyl Taurate Copolymer & Squalane & Polysorbate 60 | Simulgel NS/Seppic | 1.50 |

(continued)

| Phase D | | |
|---|---|---|
| phenoxyethanol, Methylparaben, propylparaben,Ethylparaben | Phenonip XB.Clariant | 1.00 |
| **Total** | | 100.00 |

In preparing for the erythema test, a 50 $cm^2$ test site on the backs of eleven human volunteers was subjected to seven UV exposures: the first lasting 25 seconds and each subsequent exposure lasting 25% longer than the previous, each exposure being made to a different portion of the test site. 16 to 24 hours later, the exposed areas of the human volunteers were evaluated using a chromameter to assess their MEDs (Minimal Erythematic Dose). Thereafter, each test lotion was applied at a rate of 2 $mg/cm^2$ twice a day for seven days to individual test sites measuring 4 cm x 2.5 cm on the backs of each human volunteer. Following the completion of the application protocol, the test sites and an untreated site of each volunteer were irradiated at 2x their MED. 16 to 24 hours following irradiation, the L (lightness), a (color in the red-green axis), and b (color in the blue-yellow axis) parameters on both the treated and untreated sites were measured using a Chromameter. The changes in L values (lightness), a-values (color in the red-green axis), and ITA.degree (Individual Topology Angle - COLIPA SPF test method) were determined to assess erythema. ITA.degree. was calculated using the formula:

$$ITA.degree = [Arc\ Tangent(L^*-50)/b^*)]180/3.1416$$

wherein, as noted above, L*value is lightness and b* is the color in blue-yellow axis. Table 2 presents the average L, a-value, and ITA values of the treated and untreated skin for all human volunteers prior to irradiation or UV exposure ("Pre-Irr") and following irradiation or UV exposure ("Post-Irr"). Table 2 also sets forth the delta or change in these values.

**TABLE 2**

| | Pre-Irr | Post-Irr | Δ L, AITA, Δ a-value |
|---|---|---|---|
| L-value (w/bakuchiol) | 65.69 | 66.25 | 0.56 |
| L-value (w/o) | 66.45 | 60.71 | -5.74 |
| ITA (w/bakuchiol) | 43.97 | 46.83 | -2.86 |
| ITA (w/o) | 46.05 | 36.76 | 9.29 |
| a-value (w/bakuchiol) | 8.53 | 8.38 | -0.15 |
| a-value (w/o) | 8.17 | 16.32 | 8.15 |
| * statistically significant p<0.001 | | | |

As indicated by the results shown in Table 2, the degree of erythema, as measured by a mechanical chromometer, was markedly reduced in those exposed areas that were treated with the bakuchiol containing lotion as compared those exposed areas that that were treated with the bakuchiol-free lotion. To the naked eye, erythema in the bakuchiol treated areas was barely detectable though readily visible in the areas treated with the bakuchiol-free lotion. These results were surprising inasmuch as commercial grade Bakuchiol when used in skin treatments for, e.g., psoriasis, is shown to increase erythema.

**Example 2:** Collagenase Inhibitory Activity

In order to ascertain whether other benefits may be attained by the use of meroterpenes in skin care formulations, a study was conducted on the impact, if any, the presence of bakuchiol may have on collagenase: a collagenolytic enzyme responsible for much of the collagen damage associated with UV exposure and photoaging in general. Collagenase activity was measured with an Enzcheck kit from Molecular Probes (Carlsbad, CA, USA) using quenched fluorescent gelatin and Clostridium collagenase IV, a generic metalloproteinase. Test material (aqueous solutions 1000ug/ml, 100ug/ml, 10ug/ml and 1ug/ml made from 10mg/ml stock in DMSO) was incubated in the presence of collagenase substrate - quenched fluorescin-linked gelatin and in the presence of the proteolytic enzyme. Phenanthroline, a potent metalloprotease (MP) inhibitor was used as positive control at 100ug/ml. The kinetics of the release of the digested,

fluorescent gelatin were measured at excitation/emission wavelengths of 485/530nm with Millipore Cytofluor 2350 microfluorometer. Collagenase median inhibitory concentration ($IC_{50}$) for the purified Bakuchiol was found to be ~0.1% (w/w).

These results indicate that the skin care formulations in accordance with the present invention would be expected to offer significant inhibition of collagenase as well as other damaging metalloproteiniase arising from UV exposure.

**Example 3:** Elastase Inhibitory Activity

While collaginase is perhaps the enzyme most responsible for the manifestation of skin damage due to photoaging, especially exposure to UV light, another enzyme whose activity is induced by UVA irradiation and other inflammatory processes, and which is a likely contributor to the process of photoaging, is elastase. Elastase is a protease capable of breaking down connective tissue components, especially elastin, an elastic fiber that, together with collagen, determines the mechanical properties of connective tissue. Overproduction of elastase by dermal fibroblasts has been associated with skin diseases such as cutis, laxa, acquired cutis laxa, mild dermal elastolysis, pseudoxanthoma elasticum and solar elastosis.

In an effort to ascertain whether the use of meroterpenes in skin care formulations would also affect elastase production and/or activity, a comparative study was conducted evaluating the impact of Retinol, a known skin enhancing compound, and bakuchiol on elastase activity. Stock solutions of the two actives were prepared by dissolving bakuchiol (Sytenol A, available from Sytheon Ltd of Lincoln Park, NJ, US) and Retinol 50C (available from BASF of Florham Park, NJ, US) in DMSO to a concentration of 10 mg/ml. Test solutions of each active (aqueous solutions 25 ug/ml, 5 ug/ml and 1 ug/ml made from the 10 mg/ml stock solutions in DMSO) were incubated with pure human neutrophil elastase (Elastase, Human Neutrophil, Cat. No. 324681, available from Calbiochem of San Diego, CA, US) and its substrate (Elastase Substrate VIII, Colorimetric (Suc-Ala-Ala-Ala-pNA), Cat. No. 03-32-0009, available from Calbiochem of San Diego, CA, US): each system was tested in triplicate. Controls were also employed: the first, Type 1 water served as the negative control and Elastase Inhibitor III (Cat. No. 324745, available from Calbiochem of San Diego, CA, US) served as the positive control at a concentration of 150 ug/ml. The proteolytic activity of elastase in the presence of the test materials was measured by following the generation of a chromophoric reaction product at 410 nm using a BioRad microplate reader.

The results of the comparative study are presented in Table 3. Elastase activity is presented as the percent (%) of the positive control, with the negative control (water) as the 100% of control. In the table, the results are further presented as the average of the three individual test runs. As indicated, Retinol had no statistically significant elastase inhibitory effect at all concentrations tested: indeed, at all but the lowest levels tested, elastase activity appeared greater than if no active were present at all. Conversely, the use of bakuchiol, in accordance with the practice of the present invention demonstrated a clear elastase inhibition at all concentrations tested. The positive control, Elastase Inhibitor III, showed complete inhibition of elastase activity. Based on these results, it appears that the theoretical Effective Dose 50% (ED50) for Sytenol is between 1 and 5 ug/ml. Interesting, both actives showed a reverse dose-response trend. These results, together with the results of Example 2 above indicate that the skin treatment formulations in accordance with the present invention would be expected to offer significant inhibition of skin damaging enzymatic activities, especially those associated with elastase, collagenase as well as other damaging metalloproteiniase, arising from UV exposure. In particular, it appears that the presence of the meroterpene may also offset, in whole or in part, the poor elastase inhibition or even any pro-elastase activity associated with the dermatological acids such as Retinol. Thus, substituting meroterpene for some of the retinol and other dermatological acids in conventional skin care formulations has the added benefit of enhancing elastase inhibition.

**Table 3 - Elastase Activity (% of Control)**

| Active | Control | 25 ug/ml | 5 ug/ml | 1 ug/ml |
|---|---|---|---|---|
| El III* (150 ug/ml) | 0 | | | |
| Retinol | | 416 | 138 | 87 |
| Bakuchiol | | 77 | 65 | 31 |
| Type 1 Water | 100 | | | |
| * Elastase Inhibitor III | | | | |

**Example 4:** Skin Sensitivity

Given the known sensitivity issues associated with commercial grade bakuchiol, evaluation of the skin sensitivity to the purified bakuchiol was also evaluated. Skin sensitivity was evaluated following the method cited in the reference Appraisal of the Safety of Chemicals in Food, Drugs and Cosmetics, published by The Association of Food and Drug Officials of The United States. The method employs nine inductive patching and not the ten cited in the reference under occlusive patch conditions.

Samples were prepared for evaluation by diluting the purified Bakuchiol in corn oil to a 5% concentration, with dilutions freshly prepared on each application day. 0.2 ml or 0.2 g of the diluted test material was dispensed onto the occlusive, hypoallergenic patch and the treated patch applied directly to the skin of the infrascapular regions of the back, to the right or left of the midline of each subject: one hundred and eleven subjects were employed. After application of the patch, each subject was dismissed with instructions not to wet or expose the test area to direct sunlight. The patch was removed by the subject after 24 hours. This procedure was repeated every Monday, Wednesday and Friday for three consecutive weeks until a series of nine consecutive 24 hour exposures had been made. During the test period, the test area on the subjects' backs were observed for evidence of edema or erythema just before applications two through nine and the next test date following application nine. If evidence of a reaction was found, the area of edema and/or erythema was then measured and recorded: edema being estimated by an evaluation of the skin with respect to the contour of the unaffected normal skin. The subjects were then given a 10 - 14 day rest period after which a challenge or retest dose was applied once to a previously unexposed test site. The retest dose was equivalent to any one of the original nine exposures. Reactions were scored 24 and 48 hours after application. Based on the test results, the 5% dilution in corn oil of the purified bakuchiol was determined to be a NON-PRIMARY IRRITANT and a NON-PRIMARY SENSITIZER according to the reference.

**Example 5:** Inhibition of *Propionibacterium acne*

Two series of tests were conducted to evaluate the bioefficacy of bakuchiol, salicylic acid and the combination of the two against *Propionibacterium acne.* The first evaluated the median inhibitory concentration ($IC_{50}$) of each of the two actives in order to set the dosing for the second test. The second test ascertained the bioefficacy, as represented by the zone of inhibition, of each active and their combination in relation to *P.* acne. In both sets of tests, inoculums of $1.0 \times 10^6$ cfu/ml *P.* acne were used.

Median Inhibitory Concentration ($IC_{50}$)

*P.* acne organisms were inoculated into Brain Heart Infusion (BHI) broth and incubated at 37°C for 24 hrs. Thereafter, the culture was serially diluted to a $10^{-6}$ dilution. 100 μl aliquots of the $10^{-6}$ dilution culture were then added to sterile test tubes containing 10 ml fresh BHI broth serial solutions of bakuchiol or salicylic acid. The serial solutions contained either active at from 0.5 to 50 μg/ml concentrations in 0.5 μg/ml increments, i.e., 0.5, 1.0, 1.5,..., 50.0 μg/ml. An inoculated control, free of either active, was also evaluated. The inoculated test tubes were then incubated at 37°C for 48 hrs. UV spectrophotometric readings were then made of each test tube at 610 nm and the values plotted. Based on the plots, the $IC_{50}$ values, in μg/ml, were found to be 0.6 and 29 for Bakuchiol and Salicylic acid, respectively.

Zone of Inhibition at different concentrations (Agar Well Diffusion Technique)

Agar plates were prepared by first pouring sterile molten agar into petriplates to a depth of 6mm. After allowing the agar to solidify sufficiently, a well of 10 mm diameter was prepared by carefully punching and removing a 10 mm diameter plug of agar without lifting the surrounding medium. In this experiment, all cultures used were adjusted to the turbidity of 0.5 McFarland per the standard procedure and then 0.1 ml of each culture was spread plated onto the agar plates. Appropriate controls were maintained.

Stock solutions of the actives to be tested were prepared by dissolving 1 mg of each of the actives, Sytenol A bakuchiol and salicylic acid, in separate 5 ml quantities of dimethyl sulfoxide (DMSO) at room temperature: though slight warming was employed to expedite the dissolution of the salicylic acid. These stock solutions were then diluted or mixed and diluted to produce sample solutions having the concentrations of each active as noted in Table 4 and the well of each plate filled with the appropriate solution by capillary pipette. Nothing was placed in the well of the control.

**TABLE 4**

| Sample | Active(s) & Concentration | Zone of Inhibition |
|---|---|---|
| A | Bakuchiol (0.3 μg/ml) | 35 mm |

(continued)

| Sample | Active(s) & Concentration | Zone of Inhibition |
|---|---|---|
| B | Bakuchiol (0.6 μg/ml) | 37 mm |
| C | Salicylic acid (14.5 μg/ml) | No Inhibition |
| D | Salicylic acid (29.0 μg/ml) | 12 mm |
| E | Salicylic acid (58.0 μg/ml) | 12 mm |
| F | Bakuchiol (0.3 μg/ml) + Salicylic acid (14.5 μg/ml) | 27 mm |
| G | Bakuchiol (0.6 μg/ml) + Salicylic acid (29 μg/ml) | 46 mm (additive) |
| H | Bakuchiol (0.6 μg/ml) + Salicylic acid (58 μg/ml) | 60 mm (synergistic) |

Following inoculation and addition of the test sample to the well, each plate was then covered and incubated for 48 hours at 37 °C. Following incubation, the plates were removed and the zones of inhibition recorded and tabulated. As seen in Table 4, use of the median inhibitory concentrations ($IC_{50}$) of each active (Samples B and D) successfully inhibited growth of the P. acne: though even lower levels (one-half the $IC_{50}$) of the bakuchiol by itself (Sample A) also showed excellent inhibition. However, a comparably lower level of the salicylic acid (Sample C) showed no inhibitory effect. When both actives were present at one-half the median inhibitory concentration, inhibition was seen (Sample F); however, it seemed that the apparent ineffectiveness of the salicylic acid at this level appeared to offset or counteract the effectiveness of the bakuchiol. Consequently, at least for salicylic acid, at least one-half and preferably at least the median inhibitory concentration of the dermatological acid should be used. When both actives were present at their median inhibitory concentration (Sample G), the bioefficacy was not detrimental, nor merely overlapping (as might have been suggested based on Sample F), but were additive. Even more surprising was the finding that the use of higher levels of the salicylic acid, twice the $IC_{50}$, with the $IC_{50}$ level of bakuchiol, (Sample H) produced a synergistic response even though the higher level of salicylic acid did not provide any improvement over the IC50 level (Sample E). Regardless, the results show excellent bioefficacy of the inventive combination of the present invention.

**Example 6A:** Anti-acne Clinical Study - Bakuchiol/Salicylic Acid

Four formulations were evaluated in a clinical setting as anti-acne lotions. Three of the four formulations had the following actives: (1) bakuchiol (1%), (2) bakuchiol (1%) and salicylic acid (2%), and (3) salicylic acid (2%): the last formulation was a placebo containing no active. The specific formulations were as presented in Table 5.

These formulations were prepared as follows: The components of each of Phase A and Phase B are separately combined and heated to 70-75°C. Phase A was added to Phase B while stirring. After mixing well, Phase A/B was homogenized allowing it to reach ~ 40 °C. The Phase C components were then mixed with slight warming ~ 40 °C and, once combined, added to Phase A/B. Then the emulsion was cooled down to room temperature while stirring. In the case of Salicylic acid and Salicylic acid + Bakuchiol, Phase D was added while stirring until uniform. pH was adjusted with Triethanol amine to ~5 and the composition subsequently cooled down to room temperature while stirring.

Each formulation was tested on 15 volunteer subjects, each having moderate to severe acne (but no more than twenty-five (25) facial lesions characterized as predominantly open and closed comedones). The testing was conducted over a period of four weeks with the volunteer subject's performing a self-assessment scoring. Prior to admittance to the study, each volunteer subject was examined by a Dermatologist. Facial skin condition, including actual counts of the non-inflammatory and inflammatory lesions, was recorded separately for the right and left sides of the face. Date of onset of last menstruation in case of female subjects was also recorded. Severity of comedones/acne was determined by following categories.

Grade I (mild): Less than 10 comedones (including open and closed blackheads; micropapules and whiteheads) and/or inflammatory paulpustular lesions on one or both sides of face.

Grade II (moderate): 10-25 comedones (including open and closed blackheads; micropapules and whiteheads) and/or inflammatory paulpustilar lesions on one or both sides of face.

Grade III (severe): More than 25 comedones (including open and closed blackheads; micropapules and whiteheads) and/or inflammatory paulpustular lesions on or both sides of face.

**TABLE 5**

| Ingredient | Trade Name / Supplier | Weight % | | | |
|---|---|---|---|---|---|
| | | Bakuchiol | Salicylic acid | Bakuchiol+ Salicylic acid | Placebo |
| **Phase A** | | | | | |
| Glyceryl stearate and PEG-100 | Arlacel 165/Uniema | 1.5 | 1.5 | 1.5 | 1.5 |
| Arachidyl alcohol, Behenyl alcohol, Arachidyl glucoside | Montanov 202/Seppic | 4.00 | 4.00 | 4.00 | 4.00 |
| Dimethyl isosorbide | Arlasolve DMI/ Uniqema | 3.00 | - | - | 3.00 |
| Isohexadecane | Permethyl 101A/ Presperse | 8.00 | 8.00 | 800 | 8.00 |
| Dimethicone | Dow Corning 200, 100 cst/Dow Corning | 2.00 | 2.00 | 2.00 | 2.00 |
| Bakuchiol | Sytenol® A/ Sytheon | 1.00 | - | 1.00 | - |
| **Phase B** | | | | | |
| Water | | QS to 100 | QS 100 | QS 100 | QS 100 |
| Propylene Glycol | Propylene glycol/ Lyondell | 2.00 | 2.00 | | 2.00 |
| Pentylene Glycol | Hydrolite-5/Symrise | 3.00 | - | - | 3.00 |
| Xanthan Gum | Vanzan NF/ Vanderbilt | 0.25 | 0.25 | 0.25 | 0.25 |
| **Phase C** | | | | | |
| Cyclomethicone | Dow Corning 344/Dow Corning | 4.00 | 4.00 | 4.00 | 4.00 |
| Hydroxyethylacrylat e (and) sodium acryloyldimethyl taurate copolymer | Sepinove EMT 10/Seppic | 1.00 | 1.00 | 1.00 | 1.00 |
| Phenoxyethanol, Methylparaben, Propylparaben, Ethylparaben | Phenonip XB.Clariant | 1.00 | 1.00 | 1.00 | 1.00 |
| **Phase D** | | | | | |
| Pentylene glycol | Hydrolite-5/Symrise | - | 3.00 | 3.00 | - |
| Dimethyl isosorbide | Arlasolve DMI/ Uniqema | - | 3.00 | 3.00 | - |
| Salicylic acid | | - | 2.00 | 2.00 | - |
| **Total** | | 100.00 | 100.00 | 100.00 | 100.00 |

Each lotion was applied twice daily in an amount sufficient to provide a light coating to the face. The results of the study, presented as the realized % reduction in acne, is summarized in the Table 6.

**TABLE 6**

| Group | Lotion | No. of volunteers | Percentage reduction in Acne | |
|---|---|---|---|---|
| | | | After 2 weeks | After 4 weeks |
| A | Lotion 1% Bakuchiol | 15 | - 33 | - 42 |
| B | Lotion 2% Salicylic Acid | 15 | -17 | - 36 |
| C | Lotion 1% Bakuchiol & 2% Salicylic Acid | 15 | - 35 | - 52 |
| D | Control | 15 | - 5 | - 5 |

No adverse reactions were encountered by any of the volunteer subjects in any of the four groups. Volunteer subjects in Groups A and B recorded their comments as good anti acne product leaving the skin soft and expressed a desire to continue using the products. No sun sensitivity was reported by any volunteer subject of Group A, though a few volunteer subjects of Group B did note a minor increase in sun sensitivity during the study period. Volunteers in Group C recorded their comments as very good anti acne product increasing fairness (skin lightening) and described the lotion as giving a sense of freshness and being pleasant to use. All volunteer subjects of Group C expressed a strong willingness to continue using the product. Volunteer subjects in Group D recorded their comments as good skin softening cream but with very little anti acne activity.

**Example 6B:** Anti-acne Clinical Study - Bakuchiol/Retinoic Acid

A study similar to that of Example 6A is contemplated with the exception that 0.1% retinoic acid (also referred to as tretinoin) is substituted for the salicylic acid. It is anticipated that the results will mimic those found with the prior study. Of course, when electing to employ retinoic acid, it is important to keep in mind that, while very efficacious, it is best to minimize, if not eliminate the use of retinoic acid in light of recent findings of a connection between retinoic acid and cancer: at least until such findings are sufficiently contradicted. Regardless, the synergy of the present invention is, in this case, especially desirable as it enables one to minimize the amount of retinoic acid while still benefiting from its attributes.

**Example 7:** As further support and exemplification of various skin treatment compositions and cosmetic products according to the present invention, there are provided the following inventive formulations:

Example 7A: Anti-acne lotion with 1 % Bakuchiol and 2% Salicylic acid

| INCI Name | Trade Name / Supplier | % W/W |
|---|---|---|
| **Phase A** | | |
| Water (demineralized) | | 63.25 |
| Disodium EDTA | | 0.10 |
| Propylene Glycol | | 2.00 |
| Sorbitol | Sorbo (70% soln.)/Uniqema | 2.00 |
| Sodium Lauryl Sulfate | Stepanol ME-Dry/Stepan | 0.15 |
| **Phase B** | | |
| Glyceryl Stearate | Tegin M/Goldschmidt | 5.00 |
| Stearic acid | Emersol 132/Cognis | 1.00 |
| Persea Gratissima (Avocado) oil Unsaponifiables | Crodarom Avocadin/Croda | 15.00 |
| Beeswax | White Bleached NF Beeswax Prills/Ross | 1.50 |
| Bakuchiol | Sytenol™ A / Sytheon, Present Invention | 1.00 |
| **Phase C** | | |
| Salicylic acid | | 2.00 |
| Pentylene Glycol | Hydrolite-5 / Symrise | 3.00 |
| Dimethyl Isosorbide | Arlasolve DMI / Uniqema | 3.00 |

(continued)

| | | |
|---|---|---|
| **Phase D** | | |
| Propylene glycol, DMDM Hydantoin, Methylparaben | Paragon/McIntyre | 1.00 |
| **Total** | | 100.00 |

Procedure: The components of each of Phase A and Phase B are separately combined and heated to 70-75°C. Phase A is then added to Phase B while stirring. After mixing well, Phase A/B is homogenized and allowed it to reach ~ 40 °C. The Phase C components are mixed with slight warming ~ 40 °C and then added to Phase A/B. The pH is adjusted with triethanolamine to ~4.5. Thereafter, Phase D is added while stirring until a uniform composition is achieved.

Example 7B: Anti-acne Lotion with 1 % *Psoralea corylifolia* extract containing 65% Bakuchiol + glycolic acid

| INCI Name | Trade Name / Supplier | % W/W |
|---|---|---|
| **Phase A-1** | | |
| Water (demineralized) | | 61.18 |
| Disodium EDTA | | 0.05 |
| Propylene Glycol | | 5.00 |
| **Phase A-2** | | |
| Xantham Gum | Vanzan NF/Vanderbilt | 0.25 |
| Magnesium aluminum stearate | Veegum Ultra granules/Vanderbilt | 0.40 |
| **Phase B** | | |
| Cetearyl alcohol and cetearyl glucoside | Montanov 68/Seppic | 7.00 |
| Apricot kernel oil | Lipovol P/Liop | 10.00 |
| Octyl stearate | Cetiol 868/Cognis | 3.00 |
| Dimethicone | Dow Corning 200 fluid 10cst/Dow Corning | 6.00 |
| ***Psoralea corylifolia* extract containing 65% Bakuchiol** | **Present Invention** | **2.00** |
| **Phase C** | | |
| Glycolic acid | | 4.00 |
| Triethanolamine | TEA 99%/Union Carbide | 0.12 |
| **Phase D** | | |
| Phenoxyethanol, Isopropylparaben, Isobutylparaben, butylparaben | Liquapar PE/Sutton | 1.00 |
| Total | | 100.00 |

Procedure: The ingredients of Phases A-1 and A-2 are separately combined and then phase A-2 is dispersed in phase A-1 and the mixture heated to 70-75°C. The components of phase B are combined and heated to 70-75°C. Thereafter, Phase B is added to the combined Phase A-1/A-2 while stirring. The mixture is homogenized until the mixture cools to 60°C. Glycolic acid of Phase C is then added to the Phase A/B under homogenization. The pH is then adjusted to ~4.0 using triethanolamine. Finally, Phase D is added and the combination mixed until a uniform composition is achieved.

Example 7C: Anti-acne lotion with 0.5 % Bakuchiol or Corylifolin + Retinoic acid

| INCI Name | Trade Name / Supplier | % W/W |
|---|---|---|
| **Phase A-1** | | |
| Water (demineralized) | | 63.70 |
| Disodium EDTA | | 0.05 |
| Propylene Glycol | | 5.00 |
| **Phase A-2** | | |
| Xantham Gum | Vanzan NF/Vanderbilt | 0.20 |
| **Phase B** | | |
| PEG-6 stearate, ceteth-20, glyceryl stearate, steareth-20, stearic acid | Tefose 2561/Gattefosse | 10.00 |
| Stearic Acid | Emersol 132/Cognis | 1.00 |
| Hydrogenated castor oil | Cutina HR/Cognis | 1.00 |
| Octyldodecyl myristate | M.O.D./Gattefosse | 8.00 |
| Dimethicone | Dow Corning 200, 50cst/Dow Corning | 4.00 |
| Phenyltrimethicone | Dow Corning 556 Wax/Dow Corning | 2.00 |
| **Phase C** | | |
| Sweet Almond oil | Cropure Almond/Croda | 3.00 |
| Bakuchiol or Corylifolin | Present Invention | 1.00 |
| Retinoic acid | | 0.05 |
| **Phase E** | | |
| Phenoxyethanol, Isopropylparaben, Isobutylparaben, butylparaben | Liquapar PE/Sutton | 1.00 |
| Total | | 100.00 |

Procedure: The ingredients of Phases A-1 and A-2 are separately combined and then phase A-2 is dispersed in phase A-1 and the mixture heated to 70-75°C. The components of phase B are combined and heated to 70-75°C. Phase B is then added to the combined Phase A-1/A-2 while stirring. The mixture is homogenized until the mixture cools to 40°C. Thereafter, Phase C is added at 40°C. Finally, Phase E is added and the combination mixed until uniform. This process is conducted in a dark room to avoid degradation of Retinoic acid.

Example 7D: Chemical Peel with 10% Glycolic acid and 1% Bakuchiol

| INCI Name | Trade Name / Supplier | % W/W |
|---|---|---|
| **Phase A** | | |
| Water | | 79.25 |
| Glycolic acid | | 10.00 |
| Glycerin (99.7%) | Emery 916 / Cognis | 3.50 |
| Hydroxyethylcellulose | Cellosize QP 4400 H / Amerchol | 2.00 |
| **Phase B** | | |
| Bakuchiol | Sytenol® A / Sytheon / Present invention | 1.00 |
| Polyacrylamide, C 13-13 isoparaffin, Laureth-7 | Sepigel 305 / Seppic | 2.50 |
| **Phase C** | | |
| Triethaolamine (99%) | | 0.75 |

(continued)

| | | |
|---|---|---|
| **Phase D** | | |
| Phenoxyethanol, Isopropylparaben, Isobutylparaben, butylparaben | Liquapar PE/Sutton | 1.00 |
| Total | | 100.00 |

Procedure: The hydroxyethlcellulose of Phase A is dispersed in water at room temperature. After about 10 min, the mixture is heated to ~ 60°C to improve solution process in water. The combination is allowed to cool to about 40 C, following which Glycerin and Glycolic acid are added while stirring. Separately, the components of Phase B are combined while stirring. Thereafter, Phase B is added to Phase A while stirring and the combination then homogenized until uniform. Thereafter, Phase C is added to adjust the pH to about 3.5 and the combination gently homogenized until uniform. Phase D is then added and the formulation homogenized until uniform.

Example 7E: Professional Chemical Peel with 30% Glycolic acid and Bakuchiol

| **INCI Name** | **Trade Name / Supplier** | **% W/W** |
|---|---|---|
| **Phase A** | | |
| Water | | 59.25 |
| Glycolic acid | | 30.00 |
| Glycerin (99.7%) | Emery 916 / Cognis | 3.50 |
| Hydroxyethylcellulose | Cellosize QP 4400 H / Amerchol | 2.00 |
| **Phase B** | | |
| Bakuchiol | Sytenol® A / Sytheon / Present invention | 1.00 |
| Polyacrylamide, C 13-13 isoparaffin, Laureth-7 | Sepigel 305 / Seppic | 2.50 |
| **Phase C** | | |
| Triethaolamine (99%) | | 0.75 |
| **Phase D** | | |
| Phenoxyethanol, Isopropylparaben, Isobutylparaben, butylparaben | Liquapar PE/Sutton | 1.00 |
| Total | | 100.00 |

Procedure: The hydroxyethlcellulose of Phase A is dispersed in water at room temperature. After about 10 min, the mixture is heated to ~ 60 C to improve solution processing. The mixture is allowed to cool to about 40°C, following which Glycerin and Glycolic acid are added while stirring. Separately, the components of Phase B are combined while stirring. Thereafter, Phase B is added to Phase A while stirring and the combination then homogenized until uniform. Thereafter, Phase C is added to adjust the pH to about 3.5 and the combination gently homogenized until uniform. Phase D is then added and the composition homogenized until uniform.

Example 7F: Skin Cleansing Cream

| **INCI Name** | **Trade Name / Supplier** | **% W/W** |
|---|---|---|
| **Phase A** | | |
| Borax | | 0.60 |
| Water | | 27.60 |
| Polyquaternium-24 and Hyaluronic acid | Biocare Polymer HA-24 / Amerchol | 3.80 |

(continued)

| | | |
|---|---|---|
| **Phase B** | | |
| Hydroxylated Lanolin | Ohlan / Amerchol | 3.00 |
| Isopropyl lanolate | Amerlate P/Amerchol | 2.00 |
| Bakuchiol | Sytenol® A / Sytheon / Present invention | 1.00 |
| Beeswax | | 10.00 |
| Mineral Oil, 80-90 vis | | 44.00 |
| Glycerl Stearate, PEG-100 Stearate | Arlacel 165 / Uniqema | 2.00 |
| Ozokerite | | 5.00 |
| **Phase C** | | |
| Phenoxyethanol, Isopropylparaben, Isobutylparaben, butylparaben | Liquapar PE/Sutton | 1.00 |
| Total | | 100.00 |

Procedure: The Polyquaternium 24 and Hyaluronic acid of Phase A are added to water at room temperature and mixed thoroughly until dispersed at 45 to 50 °C. Borax is then added and the combined formulation heated to ~ 80 °C. Phase B components were mixed separately while stirring and temperature raised to about 70 °C. Thereafter, Phase A is added to Phase B while stirring and the combination then homogenized for 3-4 minutes and the mixture is then allowed to cool to about 40 °C under stirring. Thereafter, Phase C is added and the combination mixed until uniform and the mixture cools to about 35 °C.

Example 7G: Anti-aging Lotion

| INCI Name | Trade Name / Supplier | % W/W |
|---|---|---|
| **Phase A-1** | | |
| Water | | 58.25 |
| Disodium EDTA | | 0.05 |
| Propylene glycol | | 5.00 |
| **Phase A-2** | | |
| Xantham gum | Vanzan NF / Vanderbilt | 0.20 |
| **Phase B** | | |
| PEG-s stearate, ceteth-20, glyceryl stearate, steareth-20, stearic acid | Tefose 2561 / Gattefosse | 10.00 |
| Stearic acid | Emersol 132 / Cognis | 1.00 |
| Hydrogenated castor oil | Cutina HR / Cognis | 1.00 |
| Octyldodecyl myristate | M.O.D / Gattefosse | 8.00 |
| Dimethicone | Dow Corning 556 wax / Dow Corning | 4.00 |
| Phenyltrimethicone | Dow Corning 556 wax / Dow Corning | 2.00 |
| Sweet Almond oil | Cropure Almond / Croda | 3.00 |
| Bakuchiol | Sytenol® A / Sytheon, Present Invention | 1.00 |
| **Phase C** | | |
| Phyllanthus emblica fruit extract | Emblica® / EMD Chemicals | 0.50 |
| Water | | 5.00 |
| **Phase D** | | |
| Phenoxyethanol, Isopropylparaben, Isobutylparaben, butylparaben | Liquapar PE/Sutton | 1.00 |

(continued)

| | |
|---|---|
| **Phase D** | |
| Total | 100.00 |

Procedure: The ingredients of Phases A-1 and A-2 are separately combined and then Phase A-2 is dispersed in Phase A-1 and the mixture heated to 70-75°C. The components of Phase B are separately combined and heated to 70-75°C. Thereafter, Phase B is added to the combined Phase A-1/A-2 while stirring. The mixture is homogenized for 3-4 minutes and allowed to cool to 60°C under stirring. Phase C is then added to the Phase A/B while stirring at ~ 40 °C. Finally, Phase D is added and the combination mixed until uniform. The pH of this formulation is 4.0.

Example 7H: Sunscreen Lotion

| INCI Name | Trade Name / Supplier | % W/W |
|---|---|---|
| **Phase A-1** | | |
| Water | | 58.13 |
| Disodium EDTA | | 0.05 |
| Propylene glycol | | 3.00 |
| **Phase A-2** | | |
| Xantham gum | Vanzan NF / Vanderbilt | 0.20 |
| **Phase B** | | |
| Glyceryl Stearate, PEG-100 Stearate | Emolium Delta / Gattefosse | 2.50 |
| Cetyl Alcohol | Crodacol C-70 / Cognis | 1.50 |
| Shea Butter | Cetiol SB-45 / Cognis | 1.50 |
| Stearic acid | Emersol 132 / Cognis | 1.00 |
| Dimethicone | DC 200 fluid / 100 cst / Dow Corning | 1.00 |
| PVP/Eicosene Copolymer | Gannes V-220 / ISP | 1.00 |
| Phenylethyl Benzoate | X-Tend 226 / ISP | 1.00 |
| Butylmethoxydibenzoylmethane | Eusoelx 9020 / EMD | 2.00 |
| Homosalate | Eusolex HMS / EMD | 15.00 |
| Octisalate | Eusolex OS / EMD | 5.00 |
| Diethyhexyl syringylidene malonate | Oxynex ST / EMD | 2.00 |
| Bakuchiol | Sytenol®A / Sytheon | 0.50 |
| **Phase C** | | |
| Ammonium Acryloyldimethyltaurate/ VP Copolymer | Arsitoflex AVC / Clariant | 0.60 |
| Cyclomethicone | DC 345 / Dow Corning | 3.00 |
| **Phase D** | | |
| Aminomethylpropanol | AMP-95 | 0.02 |
| **Phase E** | | |
| Phenoxyethanol, Isopropylparaben, Isobutylparaben, butylparaben | Liquapar PE/Sutton | 1.00 |
| Total | | 100.00 |

Procedure: The ingredients of Phases A-1 and A-2 are separately combined and then Phase A-2 is dispersed in Phase A-1 and the mixture heated to 70-75°C. The components of Phase B are separately combined and heated to 70-75°C. Thereafter, Phase B is added to the combined Phase A-1/A-2 while stirring. The mixture is homogenized for 2-3 minutes and the mixture cooled to 45°C. Phase C is then added while stirring. Thereafter, Phases D & E are added under stirring until uniform. The pH of this formulation is about 5.9.

Without further elaboration, it is believed that one skilled in the art, using the preceding description, can utilize the teachings set forth herein to its fullest extent and can make various changes and modifications of the invention to adapt it to various usages and conditions. The preceding preferred specific embodiments are, therefore, to be construed as merely illustrative, and not limitative of the remainder of the disclosure in any way whatsoever.

## Claims

1. A skin treatment composition comprising a) salicylic acid; b) bakuchiol and (c) a dermatological acceptable carrier, wherein the bakuchiol (b) is (i) present as a purified or isolated material having a purity of at least 60% w/w or as a purified plant extract containing 1 to about 70% or more by weight bakuchiol based on the total weight of the extract and (ii) contains less than 0.1 wt% of furocoumarins and wherein the amount of bakuchiol present in the composition is at least its median inhibitory concentration on *Propionibacterium acne* (MIC) amount and the amount of salicylic acid present in the composition is at least twice its MIC amount.

2. The composition of claim 1 wherein the bakuchiol is present in an amount of 0.1 to 10% based on the total composition.

3. The composition of claim 1 wherein the bakuchiol is present in an amount of 0.5 to 5% based on the total composition.

4. The composition of any of claims 1 to 3 wherein the bakuchiol is a purified or isolated material having a purity of at least 95% w/w.

5. The composition of any of claims 1 to 3 wherein the bakuchiol is added as a bakuchiol enriched plant extract derived from *Psoralea corylifolia, P. grandulosa,* or *Otholibium pubescens* and containing from 85 to 99.99% by weight of the meroterpene.

6. The composition of any of claims 1 to 5 wherein the salicylic acid is present in an amount of up to 10% by weight based on the total composition.

7. The composition of any of claims 1 to 5 wherein the salicylic acid is present in an amount of from 10 to 70% by weight based on the total composition.

8. The composition of any of claims 1 to 5 wherein the salicylic acid is present in an amount of from 20 to 30% by weight based on the total composition.

9. The composition of any of claims 1 to 5 wherein the salicylic acid is present in an amount of from 50 to 70% by weight based on the total composition.

10. The composition of any of claims 1 to 9 further comprising one or more ingredients are selected from the group consisting of antioxidants, vitamins, anti-inflammatory agents, moisturizers, emollients, humectants, and mixtures thereof.

## Patentansprüche

1. Hautbehandlungszusammensetzung, die a) Salizylsäure; b) Bakuchiol und (c) einen dermatologisch zulässigen Trägerstoff aufweist, wobei das Bakuchiol (b) (i) als ein gereinigtes oder isoliertes Material mit einer Reinheit von mindestens 60% w/w oder als ein gereinigter Pflanzenextrakt vorhanden ist, der 1 bis ungefähr 70 oder mehr Gewichtsprozent Bakuchiol basierend auf dem Gesamtgewicht des Extraktes enthält, und (ii) weniger als 0,1 Gewichtsprozent Furocoumarin enthält, und wobei die Menge an Bakuchiol, die in der Zusammensetzung vorhanden ist, zumindest seine Menge für mittlere inhibitorische Konzentration (MIC = median inhibitory concentration) auf Propionibacterium Acne ist, und wobei die Menge an Salizylsäure, die in der Zusammensetzung vorliegt, zumindest zweimal ihre MIC-Menge ist.

2. Zusammensetzung nach Anspruch 1, wobei das Bakuchiol in einer Menge von 0,1 bis 10% basierend auf der Gesamtzusammensetzung vorhanden ist.

3. Zusammensetzung nach Anspruch 1, wobei das Bakuchiol in einer Menge von 0,5 bis 5% basierend auf der Gesamtzusammensetzung vorhanden ist.

4. Zusammensetzung nach einem der Ansprüche 1 bis 3, wobei das Bakuchiol gereinigtes bzw. isoliertes Material ist, welches eine Reinheit von mindestens 95% w/w hat.

5. Zusammensetzung nach einem der Ansprüche 1 bis 3, wobei das Bakuchiol als ein bakuchiol-angereicherter Pflanzenextrakt zugegeben ist, der aus Psoralea Corylifolia, P. Grandulosa oder Otholibium Pubescens gewonnen wurde und von 85 bis 99,99 Gewichtsprozent des Meroterpens enthält.

6. Zusammensetzung nach einem der Ansprüche 1 bis 5, wobei die Salizylsäure in einer Menge von bis zu 10 Gewichtsprozent basierend auf der Gesamtzusammensetzung vorhanden ist.

7. Zusammensetzung nach einem der Ansprüche 1 bis 5, wobei die Salizylsäure in einer Menge von 10 bis 70 Gewichtsprozent basierend auf der Gesamtzusammensetzung vorhanden ist.

8. Zusammensetzung nach einem der Ansprüche 1 bis 5, wobei die Salizylsäure in einer Menge von 20 bis 30 Gewichtsprozent basierend auf der Gesamtzusammensetzung vorhanden ist.

9. Zusammensetzung nach einem der Ansprüche 1 bis 5, wobei die Salizylsäure in einer Menge von 50 bis 70 Gewichtsprozent basierend auf der Gesamtzusammensetzung vorhanden ist.

10. Zusammensetzung nach einem der Ansprüche 1 bis 9, die weiter einen oder mehrere Inhaltsstoffe aufweist, die aus der Gruppe ausgewählt sind, welche aus Antioxidantien, Vitaminen, entzündungshemmenden Mitteln, Feuchtigkeitsspendern, Emolliens, Feuchthaltemittel und Mischungen davon besteht.

**Revendications**

1. Composition de traitement de la peau comprenant a) de l'acide salicylique ; b) du bakuchiol et (c) un support acceptable du point de vue dermatologique, dans laquelle le bakuchiol (b) est (i) présent sous forme d'un matériau purifié ou isolé ayant une pureté d'au moins 60 % en poids ou d'un extrait de plante purifié contenant de 1 à environ 70 % ou plus en poids de bakuchiol par rapport au poids total de l'extrait et (ii) contient moins de 0,1 % en poids de furocoumarines et dans laquelle la quantité de bakuchiol présente dans la composition est au moins sa concentration inhibitrice médiane sur une quantité de *Propionibacterium acne* (MIC) et la quantité d'acide salicylique présente dans la composition est au moins le double de sa quantité MIC.

2. Composition selon la revendication 1, dans laquelle le bakuchiol est présent dans une quantité de 0,1 à 10 % par rapport à la composition totale.

3. Composition selon la revendication 1, dans laquelle le bakuchiol est présent dans une quantité de 0,5 à 5 % par rapport à la composition totale.

4. Composition selon l'une quelconque des revendications 1 à 3, dans laquelle le bakuchiol est un matériau purifié ou isolé ayant une pureté d'au moins 95 % en poids.

5. Composition selon l'une quelconque des revendications 1 à 3, dans laquelle le bakuchiol est ajouté sous la forme d'un extrait de plante enrichi en bakuchiol dérivé de *Psoralea corylifolia, P. grandulosa* ou *Otholibium pubescens* et contenant de 85 à 99,99 % en poids du méroterpène.

6. Composition selon l'une quelconque des revendications 1 à 5, dans laquelle l'acide salicylique est présent dans une quantité jusqu'à 10 % en poids par rapport à la composition totale.

7. Composition selon l'une quelconque des revendications 1 à 5, dans laquelle l'acide salicylique est présent dans une quantité de 10 à 70 % en poids par rapport à la composition totale.

8. Composition selon l'une quelconque des revendications 1 à 5, dans laquelle l'acide salicylique est présent dans une quantité de 20 à 30 % en poids par rapport à la composition totale.

**9.** Composition selon l'une quelconque des revendications 1 à 5, dans laquelle l'acide salicylique est présent dans une quantité de 50 à 70 % en poids par rapport à la composition totale.

**10.** Composition selon l'une quelconque des revendications 1 à 9, comprenant en outre un ou plusieurs ingrédients sélectionnés dans le groupe comprenant antioxydants, vitamines, agents anti-inflammatoires, hydratants, émollients, agents humectants et leurs mélanges.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2005048008 A
- IN 00570KOL2005
- US 20060251749 A, Jia
- US 7186404 B, Gonzalez
- US 7175834 B, Aust
- US 7172754 B, Roseaver
- US 7175835 B, Simoulidis
- US 7101536 B, Mongiat
- US 7078022 B, Maniscalco
- US 5175340 A, Forestier
- US 5567418 A
- US 5538716 A
- US 5951968 A
- US 5670140 A, Deflandre
- US 7150876 B, Chaudhuri
- US 6831191 B
- US 6602515 B
- US 7166273 B
- US 6936735 B
- US 6699463 B
- US 6165450 A, Chaudhuri
- US 6962692 B, Bonda
- US 5830441 A, Wang
- US 6124268 A, Ghosal
- US 20050048008 A1, Gupta
- US 4185100 A, Rovee
- US 4338293 A, Holick
- WO 11803188 A
- US 4693623 A, Schwartzman
- US 4620648 A, Schwartzman
- US 3669323 A, Harker
- US 3418055 A, Schwartzman
- US 3410645 A, Schwartzman
- US 4891228 A, Thaman
- US 4891227 A, Thaman


### Non-patent literature cited in the description


- **HALLIDAY, G. M. et al.** Topical retinoic acid enhances, and a dark tan protects, from sub-dermal solar-simulated photocarcinogenesis. *J. Invest. Dermatol.,* 2000, vol. 114, 923
- **CN BACKHOUSE ; CL DELPORTE ; RE NEGRETE ; S ERAZO ; A ZUNIGA ; A PINTO ; BK CASSELS.** *J Ethnopharmacology,* 2001, vol. 78 (1), 27-31
- **H HARAGUCHI ; J INOUYE ; Y TAMARA ; K MIZUTANI.** *Planta Medica,* 2000, vol. 66 (6), 569-571
- **JM KRENISKY ; J LUO ; MJ REED ; JR CARNEY.** *Biol Pharm Bull,* 1999, vol. 22 (10), 1137-1140
- **H KATSURA ; R TSUKIYAMA ; A SUZUKI ; M KOBAYASHI.** *Antimicrobial Agents and Chemotherapy,* 2001, vol. 45 (11), 3009-3013
- **S ADHIKARI ; R JOSHI ; BS PATRO ; TK GHANTY ; GJ CHINTALWAR ; A SHARMA ; S CHATTOPADHAYA ; T MUKHERJEE.** *Chem Res Toxicol,* 2003, vol. 16, 1062-1069
- **JB PERALES ; NF MAKINO ; DL VAN VRANKEN.** *J Org Chem,* 2002, vol. 67, 6711-6717
- ICI Handbook. 1612-1613
- **LAW et al.** *Br. J. Pharmac.,* 1977, vol. 59 (4), 591-597
- **KAIDBEY.** *J. Invest. Dermatoloy,* 1976, vol. 66, 153-156
- **GRUBER et al.** *Clinical Pharm. and Therapeut.,* 1971, vol. 13 (1), 109-113